# EUROPEAN PATENT SPECIFICATION

(11) **EP 3 030 674 B1**
(45) Date of publication and mention of the grant of the patent: **14.04.2021**
(21) Application number: 14750187.8
(22) Date of filing: 06.08.2014
(51) Int. Cl.: C12Q 1/6888

(54) **METHOD FOR DETECTION OF PYRETHROID RESISTANCE IN CRUSTACEANS AND OLIGONUCLEOTIDE SEQUENCES USEFUL IN DETECTION OF PYRETHROID RESISTANCE.**
VERFAHREN ZUM NACHWEIS EINER PYRETHROIDRESISTENZ IN SCHALENTIEREN UND OLIGONUKLEOTIDSEQUENZEN ZUM NACHWEIS EINER PYRETHROIDRESISTENZ
MÉTHODE POUR LA DÉTECTION DE LA RÉSISTANCE AUX PYRÉTHROÏDES CHEZ LES CRUSTACÉS ET SÉQUENCES OLIGONUCLÉOTIDIQUES UTILES DANS LA DÉTECTION DE RÉSISTANCE AUX PYRÉTHROÏDES.

(30) Priority: 06.08.2013 NO 20131075
(43) Date of publication of application: 15.06.2016
(73) Proprietor: PATOGEN AS, 6001 Ålesund (NO); Bergen Teknologioverføring AS, 5008 Bergen (NO)
(72) Inventor: NILSEN, Frank, 5006 Bergen (NO); ESPEDAL, Per Gunnar, 5006 Bergen (NO)
(74) Representative: Onsagers AS
(86) International application number: PCT/EP2014/066895
(87) International publication number: WO 2015/018861

(56) References cited:
- EP-A1- 0 615 976
- WO-A2-01/14592
- US-A1- 2007 218 476
- US-A1- 2011 166 037
- US-A1- 2011 178 283
- US-B1- 6 599 701
- VAN LEEUWEN THOMAS ET AL: "Acaricide resistance mechanisms in the two-spotted spider mite Tetranychus urticae and other important Acari: a review", INSECT BIOCHEMISTRY AND MOLECULAR BIOLOGY AUG 2010,, vol. 40, no. 8, 1 August 2010 (2010-08-01) , pages 563-572, XP002731810, ISSN: 1879-0240
- VAN LEEUWEN THOMAS ET AL: "Mitochondrial heteroplasmy and the evolution of insecticide resistance: non-Mendelian inheritance in action", NATIONAL ACADEMY OF SCIENCES. PROCEEDINGS, NATIONAL ACADEMY OF SCIENCES, UNITED STATES, vol. 105, no. 16, 22 April 2008 (2008-04-22), pages 5980-5985, XP002731811, ISSN: 1091-6490
- TJENSVOLL KJERSTI ET AL: "Sequence variation in four mitochondrial genes of the salmon louse Lepeophtheirus salmonis", DISEASES OF AQUATIC ORGANISMS, INTER-RESEARCH, DE, vol. 68, no. 3, 2 March 2006 (2006-03-02), pages 251-259, XP002731812, ISSN: 0177-5103
- DATABASE EMBL [Online] EBI; 3 February 2006 (2006-02-03), "Lepeophtheirus salmonis isolated cytb gene", XP002731859, Database accession no. AY602377
- DATABASE EMBL [Online] EBI; 3 February 2006 (2006-02-03), "Lepeophtheirus salmonis isolated cytb gene", XP002731860, Database accession no. AY602296

## Description

### Field of the invention

The present invention relates to a method for detection of single polymorphisms associated with pyrethroid resistant *Lepeophteirus salmonis,* and oligonucleotide sequences and kits useful in the method of the present invention. The present invention furthermore provides kits and reagents useful for the detection of pyrethroid resistance associated SNPs.

### Background of the invention

Infestations of the parasitic copepod *Lepeophtheirus salmonis,* commonly referred to as sea lice, represent a major challenge to commercial salmon aquaculture. Control measures have been reliant upon the use of a limited number of chemotherapeutants since the 1970's, and reduced efficacy has now been reported for the majority of these compounds. Reduced sensitivity and potential resistance to currently available medicines are constant threats to maintaining control of sea lice populations in Atlantic salmon farms. Today, resistance against most of the available treatment options is widespread in almost every region where salmonids are cultured in sea water. Double- or triple resistance, in which sea lice is resistant to more than one type of chemotherapeutant, have been verified.

Sea lice (*Lepeophtheirus salmonis* and *Caligus* spp.) are the major pathogens affecting global salmon farming industry and have a significant impact in many areas. The annual loss has recently been estimated to €300 million **(**Costello M. J. (2009). The global economic cost of sea lice to the salmonid farming industry. Journal of Fish Diseases. 32. 115-118) and the aquaculture industry relays heavily on a few chemotherapeutants for lice control. Emerging resistance development to these drugs increase the necessity to develop new treatment methods (biological, prophylactic and drugs) and tools to avoid increased loss due to sea lice and to ensure a sustainable salmon farming industry in the future. Control measures have relied upon a limited number of chemotherapeutants since the 1970s. Parasite resistance and reduced efficacy have now been reported for the majority of these compounds (Sevatdal S., Copley L., Wallace C., Jackson D., Horsberg T.E. (2005). Monitoring of the sensitivity of sea lice (Lepeophtheirus salmonis) to pyrethroids in Norway, Ireland and Scotland using bioassays and probit modeling). Aquaculture 244. 19-27). In many regions, the recent development of resistance to emamectin benzoate has resulted in heavy mortalities, significant economic impact as well as increased prevalence of other infectious diseases (Bravo S., Erranz F., Lagos C. (2009) A comparison of sea lice, Caligus rogercresseyi, fecundity in four areas in southern Chile. Journal of Fish Diseases. 32. 107-113).

Although various chemotherapeutants have been in use in the aquaculture industry for more than 30 years, it is only during the last 15 years that such use has been part of some kind of integrated pest management system. Management practices include coordinated salmon production within a defined area, use of single year class of fish, limited production period, fallowing, coordinated restocking, use of wrasse, synchronized treatments during the winter and targeting female lice to reduce the impact of settlement during the spring (Pike A., Wadsworth S. L., (2000), Sea Lice: A review. Advances in Parasitology. Academic Press. 44. 232-337).

Further controls are required to progress towards a true integrated pest management (IPM) system common to other forms of food production. One key to succeeding with a IPM is to develop tools for the management of resistance to the medicines in use (Brook K. (2009). Considerations in developing an integrated pest management program for control of sea lice on farmed salmon in Pacific Canada. Journal of Fish Diseases. 32. 59-73). So far, drug resistance in sea lice has been detected by various types of bioassays as a significant increase in EC50/LC50. Although the bioassays can detect any type of resistance to a given drug, such methods are not very accurate or sensitive.

The genomes of all organisms undergo spontaneous mutations during their continuing evolution, forming variant forms of progenitor genetic sequences. A mutation may results in an evolutionary advantage or disadvantage relative to a progenitor form or may be neutral. A variant that result in an evolutionary advantage may eventually be incorporated in many members of the species and may thus effectively become the progenitor form. Furthermore, often various variant forms survive and coexist in a species population. The coexistence of multiple forms of a genetic sequence gives rise to genetic polymorphism, including single-nucleotide polymorphisms (SNPs).

A single-nucleotide polymorphism (SNP) is a DNA sequence variation occurring when a single nucleotide - A, T, C or G - in the genome (or other shared sequence) differs between members of a biological species or paired chromosomes in an organism. For example, two DNA fragments from different individuals, AAGCCTA to AAGCTTA, contain a difference in a single nucleotide, commonly referred to as two alleles. Almost all common SNPs have only two alleles. The genomic distribution of SNPs is not homogenous; SNPs usually occur in non-coding regions more frequently than in coding regions or, in general, where natural selection is acting and fixating the allele of the SNP that constitutes the most favorable genetic adaptation (Adapted from Wikipedia).

Previously, non-mendelian inheritance of resistance by reciprocal crosses between resistant and spider mite susceptible of being resistant towards a hydrazine carbazate derivate have been linked to mutations in mitochondrial DNA of the spider mite (Van Leeuwen, T., Vanholme, B., Van Pottelberge, S., Van Nieuwenhuyse, P., Nauen, R., Tirry, L., & Denholm, I. (2008). Mitochondrial heteroplasmy and the evolution of insecticide resistance: non-Mendelian inheritance in action. Proceedings of the National Academy of Sciences of the United States of America, 105(16), 5980-5). This represents the only other known example of mutations in mitochondrial DNA linked to resistance towards an insecticide apart from the study presented here in sea lice. The spider mite however showed resistance towards a hydrazine carbazate derivate, and not towards pyrethroids as shown for in sea lice in the present application. Spidermite and sea lice are distantly related. Up to date, no SNPs consequently associated with chemotherapeutant resistance in sea lice have been reported, and particularly, no SNPs in the mitochondrial DNA has been linked to pyrethroid resistance in any species. However, in many arthropods, specific mutations in the gene coding for the voltage gated sodium channel have been reported to result in an altered function of this channel, resulting in a decreased ability for pyrethroids to interfere with its function (Zlotkin E, (1999), "The insect voltage-gated sodium channel as target of insecticides", Annu Rev Entomol. 44:429-55).

Another mechanism which has been suspected to contribute in the resistance towards pyrethroids is enhanced detoxification capability by the parasite. From other arthropods, increased activities of monooxydases and unspecific esterases have been identified as mechanisms in question. However, esterases seem to play an insignificant role in sea lice, as the parasite has a very low background activity of these enzymes. On the other hand, unspecific monooxidases seem to play a significant role, indicated by a correlation between enzyme activity and reduced sensitivity.

The mitochondrial genes COI, A6, Cytb and 16sRNA of *Lepeophtheirus salmonis* has shown to display high levels of polymorphisms (Tjensvoll et al., 2006, Diseases of Aquatic Organisms, vol, 68, pp. 251 - 259), resulting in a significant number of haplotypes. However, the prior art is silent as to whether the large number of polymorphic sites have had any effects on the characteristics of the sea lice.

Pyrethroids are one type of chemotherapeutics used for delousing of commercial salmon aquaculture sites infested with sea lice. In Norway, the synthetic deltamethrin (AlphaMax™) and cis-cypermethrin (BetaMax™) are the most widely used pyrethroids. In Scotland and Ireland, cypermethrin (ExisTM) has been commonly used. The pyrethroids affect the sodium channels thus inhibiting the transmittal of nerve impulses in the synapses. Failure of pyrethroid treatment has been reported in Norway (deltamethrin and cis-cypermethrin) since turn of the century (Sevatdal and Horsberg, 2000, Norsk Fiskeoppdrett, vol. 12, pp. 34-35), and reduced sensitivity towards pyrethroids was documented in 2003 (Sevatdal og Horsberg, 2003, Aquaqulture, vol. 218, pp. 21-31).

Efficient and sensitive methods for diagnosing resistance are crucial in order to manage and control drug resistance. Early detection of reduced sensitivity to a chemical can enable effective countermeasures to be enforced at a time point when these have a greater probability of being effective. Therefore, accurate and speedy identification of pyrethroid resistant *Lepeoptheirus salmonis* (PRLS) is crucial. Detection of PRLS prior to treatment, and the use of such analyses after treatment to evaluate treatment efficacy constitutes an important determinant for the integrated pest management (IPM) in the aquaculture industry.

### Summary of invention

The present invention is based on the surprising finding that resistance towards chemotherapeutics commonly used to combat sea lice infestation is linked to mutations in the mitochondrion genome of the sea lice *Lepeophtheirus salmonis.* More particularly, the present invention is based on the identification of novel single-nucleotide polymorphisms (SNPs) in the mitochondrion genome of the sea lice *Lepeophtheirus salmonis* shown to be involved in the resistance towards pyrethroid-based chemotherapy. Specifically, the present inventors have identified two pyrethroid resistance-associated SNPs located in the mitochondrial CoI gene (T8605C, A9035G) and three pyrethroid resistance-associated SNP in the mitochondrial Cyt b gene (C13957T, A14017G and C14065T).

It is further noted that the identification of the SNPs involved in pyrethroid resistance in the mitochondrial genome proved to be challenging due to unexpected characteristics such as numerous repeats etc. of the non-coding parts of the mitochondrial genome (D-loop).

The present invention thus provides an in vitro method for determination of pyrethroid resistance in crustaceans, comprising the steps of
a. analyzing the mitochondrial genomic material of the crustacean,
b. detecting one or more single nucleotide polymorphisms (SNP) associated with pyrethroid resistance in the mitochondrial DNA of the crustacean to be analyzed.

. The present invention furthermore provides for novel isolated sequences comprising SNPs involved in the resistance towards pyrethroid-based chemotherapy, and their use in determination of pyrethroid resistance in crustaceans. In particular, said method is useful for detection of pyrethroid resistance in copepods, in particular copepods belonging to the family Caligidae, in particular species selected from the group consisting of *Lepeophteirus salmonis, Caligus elongatus,* and *Caligus rogercresseyi.*
According to one embodiment, the present invention provides a method for detection of pyrethroid resistance in sea lice, comprising the steps of detecting single nucleotide polymorphism (SNP) associated with pyrethroid resistance in the mitochondrial genome of a sea lice to be analyzed, wherein said sea lice is resistant to pyrethroids if at least one of the following nucleotides:
i. C in position 8605;
ii. G in position 9035;
iii. T in position 13957;
iv. G in position 14017;
v. T in position 14065;
or the complementary oligonucleotide thereof, is present in the mitochondrial genome sequence of the one or more sea lice to be analyzed, and wherein the numbering of said positions is in accordance with the sequence depicted in SEQ ID No. 1. According to one embodiment, the pyrethroid resistance linked SNPs mentioned above is detected in Lepeophteirus salmonis.

According to yet another aspect, a method is provided, comprising the steps of: a) collecting sea lice from infested fish or water samples; b) isolating mitochondrial genomic material from any life stage of collected sea lice; c) determining the nucleotide polymorphic site at the positions 8605, 9035 and 14065 of the isolated mitochondrial DNA compared with of the mitochondrial nucleic acid sequence SEQ ID No. 1, wherein said sea lice is resistant to pyrethroids if at least one of the nucleotide C, G, T, G, and T (U), or a complementary oligonucleotide thereof, is present in position 8605, 9035, 13957, 14017, and 14065, respectively.

According to one embodiment of the above method, said step c) is performed using a primer selected from the group consisting of SEQ ID No. 6-15.

A method according to yet another embodiment, said step c) is performed using at least one probe selected from the group consisting of SEQ ID No. 16-20.

According to yet another embodiment, said step c) comprises nucleic acid amplification, e.g. using polymerase chain reaction.

According to yet another embodiment, said step c) is performed by contacting mitochondrial DNA sequence of the sea lice to be analyzed with a detection reagent, and determining which nucleotide is present in position 8605, 9035, 13957, 14017 and 14065.

According to yet another embodiment, said detection reagent is an oligonucleotide probe.

According to yet another embodiment, said step c) is performed using SNP specific probe hybridization, SNP specific primer extension, SPN specific amplification, sequencing, 5' nuclease digestion, molecular beacon assay, oligonucleotide ligation assay, size analysis, single-stranded conformation polymorphism analysis, denaturing gradient gel electrophoresis.

According to another embodiment of the present method of the invention, the pyrethroid is selected from the group consisting of deltamethrin, and cis-cypermethrin.

Furthermore, disclosed herein is an isolated oligonucleotide sequence comprising small nucleotide polymorphism (SNP) associated with pyrethroid resistance in crustaceans, such as copepods, in particular copepods belonging to the family Caligidae, in particular species selected from the group consisting of *Lepeophteirus salmonis, Caligus elongatus,* and *Caligus rogercresseyi,*
wherein said isolated oligonucleotide sequence comprises nucleotides that distinguishes sea lice which are resistant to pyrethroids from non-resistance to pyrethroids, and wherein the SNP are present in the mitochondrial DNA of the organism.

According to one embodiment, an isolated oligonucleotide is provided comprising a single-nucleotide polymorphism (SNP) associated with pyrethroid resistance in crustaceans, wherein said isolated oligonucleotide sequence comprises nucleotides that distinguishes crustaceans which are resistant to pyrethroids from non-resistant crustaceans, and wherein the SNPs are present in the mitochondrial DNA of the crustaceans, and wherein said isolated oligonucleotide sequence comprises at least 8 contiguous nucleotides of the sequences selected from the group consisting of SEQ ID No. 5, or complementary sequences of SEQ ID No. 5, provided that at least one SNP selected from the group of SNPs consisting of T8605C (U8605C in case of RNA), A9035G, C13957T (U13957T in case of RNA), A14017G, and C14065T (C14065U in case of RNA) is present, and wherein the numbering of said positions is in accordance with the sequence depicted in SEQ ID No. 1.

According to one embodiment, an oligonucleotide probe or oligonucleotide primer is provided comprising a oligonucleotide sequence being homologous to a fragment of an isolated oligonucleotide sequence according to the present invention, said probe or primer being specific for a mitochondrial DNA sequence of sea lice associated with pyrethroid resistance comprising at least one SNPs selected from the group consisting of T8605C and A9035G of SEQ ID No. 4, andC13957T, A14017G, and C14065T of SEQ ID 5, and wherein the numbering of said positions is in accordance with the sequence depicted in SEQ ID No. 1.

According to one embodiment, said isolated oligonucleotide sequence comprises at least 20 contiguous nucleotides.

According to yet another embodiment, an isolated oligonucleotide sequence comprising at least one single-nucleotide polymorphism (SNP) associated with pyrethroid resistance in crustaceans, wherein said isolated oligonucleotide sequence comprises nucleotides that distinguishes crustaceans which are resistant to pyrethroids from non-resistance to pyrethroids, and wherein the SNPs are present in the mitochondrial DNA of the crustaceans, and wherein said oligonucleotide sequence comprises 12 to 25 consecutive nucleotides of SEQ ID No. 4, or complementary sequences of SEQ ID No. 4, provided that at least one SNP selected from the group of SNPs consisting of T8605C (U8605C in case of RNA), A9035G, C13957T (U13957T in case of RNA), and C14065T (C14065U in case of RNA) is present in said isolated oligonucleotide sequence, and wherein the numbering of said positions is in accordance with the sequence depicted in SEQ ID No. 1.

According to yet another embodiment, the probe or primer according to the present disclosure comprising at least 8 contiguous nucleotides of SEQ ID No. 1, including at least one of the nucleotide C, G, T, G, and T (U), or a complementary oligonucleotide thereof in the position corresponding to position 8605, 9035, 13957, 14017 and 14065, respectively, of SEQ ID 1.

According to one embodiment, a probe is provided, wherein the sequence of said probe is selected from the group consisting of SEQ ID No. 16, SEQ ID No. 17, SEQ ID No. 18, and SEQ ID No. 20, and sequences having over their entire length at least 80 % sequence identity therewith, or a complementary sequence thereof.

According to one embodiment, a primer is provided, wherein the sequence of said primer is selected from the group consisting of SEQ ID No. 6, SEQ ID No. 7, SEQ ID No. 8, SEQ ID No. 9, SEQ ID No. 10, SEQ ID No. 11, SEQ ID No. 14, and SEQ ID No. 15, and sequences having over their entire length at least 80 % sequence identity therewith, or a complementary sequence thereof.

Furthermore, the present invention provides a kit for detection of pyrethroid resistance in crustaceans, such as copepods, e.g. in sea lice (Lepeophteirus salmonis), comprising a probe or primers according to the present invention.

Finally, the present disclosure provides an isolated oligonucleotide sequence comprising at least 8 contiguous nucleotides of the sequence selected from the group consisting of SEQ ID No. 4and SEQ ID No. 5, and wherein said sequence comprises at least one of the nucleotide C, G, C, A, and T (U) or a complementary oligonucleotide thereof in the position corresponding to position 8605, 9035 and 14065, and wherein the numbering of said positions is in accordance with the sequence depicted in SEQ ID No. 1.

### Figures

**Figure 1** illustrates the hybridization experiment showing that pyrethroid resistance characteristics are transmitted from female sea lice to their progenies.
**Figure 2** illustrates the experiment flow of the hybridization experiment. Two field-collected strains of lice were initially used to create two hybrid strains: *Ls*HybridG with *Ls*Gulen as its maternal strain, and *Ls*HybridV with *Ls*Vikna as its maternal strain.

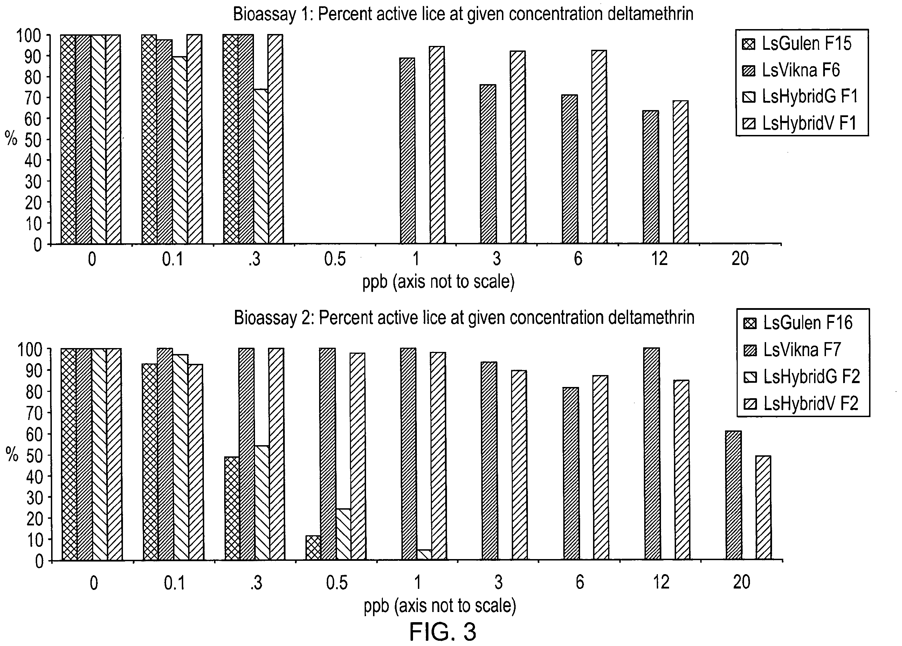

**Figure 3** show percent active lice in deltamethrin bioassays 1 and 2. In bioassay 1 the concentrations 0.5 and 20 ppb were not included, and in bioassay 2 *Ls*Gulen F7 was not tested for 20 ppb.

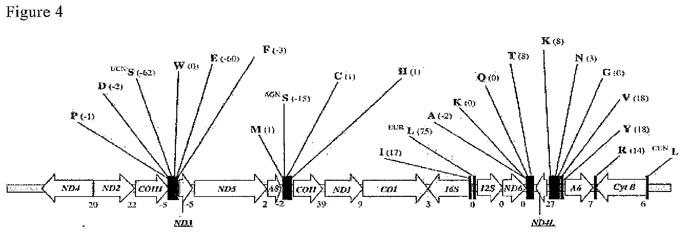

**Figure 4** shows the organization of mitochondrial DNA of *L. salmonis.*
**Figur 5** shows an alignment of mitochondria sequences from *L. salmonis.* The upper sequence is the reference sequence of the *L. salmonis* mtDNA deposited by Tjensvoll et al (2005) with NCBI accession number NC_007215 (http://www.ncbi.nlm.nih.gov/nuccore/NC_007215.1), corresponding to SEQ ID No. 1.

The start point in this sequence is in the D-loop (i.e. the non-coding region of the mtDNA). The sequence named LsGulen2 is isolated from a pyrethroid sensitive strain and is depicted in SEQ ID No. 2 and 3. The sequence named LsVikna1 is isolated from a strain resistant to pyrethroids, and is depicted in SEQ ID No. 4 and 5.

The present invention and its various embodiments will be described in more detail in the following and is defined in the appended claims.

### Detailed description of the invention

The present invention provides an in vitro method for determination of pyrethroid resistance in crustaceans, including copepods, in particular *Lepeophtheirus salmonis,* and novel SNPs, based on the surprising findings that mutations linked with resistance against pyrethroid in *Lepeophtheirus salmonis* were found in mitochondrial DNA.

Although pyrethroid resistance linked SNPs presented in the experimental data was identified in the sea lice species *Lepeophtheirus salmonis,* the skilled person will acknowledge, based on the teaching herein, that the present method and the present oligonucleotides may be used to determine pyrethroid resistance in crustaceans, in particular copepods, in particular copepods belonging to the family Caligidae. In particular, it is to be understood that the present method and the present oligonucleotides may be used to determine pyrethroid resistance in copepods affecting farmed fish, such as fish belonging to the family *Salmonidae.* According to one embodiment, the present method and present oligonucleotides are useful for detection of pyrethroid resistance in copepod selected from the group consisting of *Lepeophteirus salmonis, Caligus elongatus,* and *Caligus rogercresseyi.*

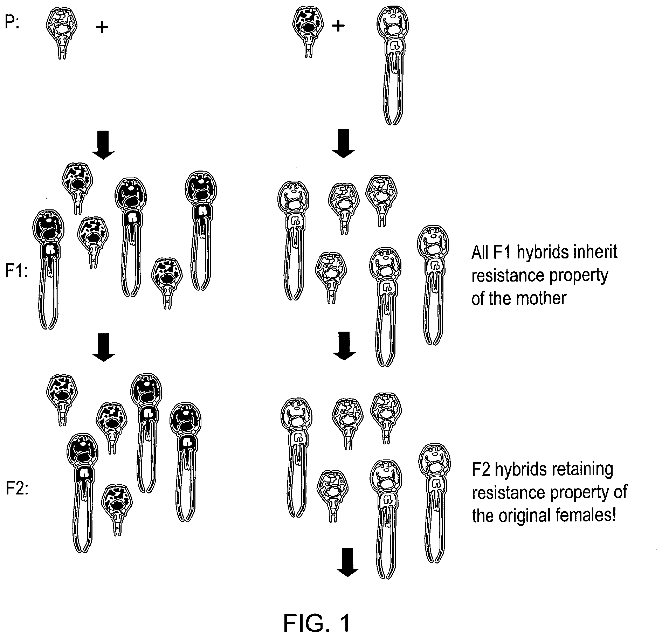

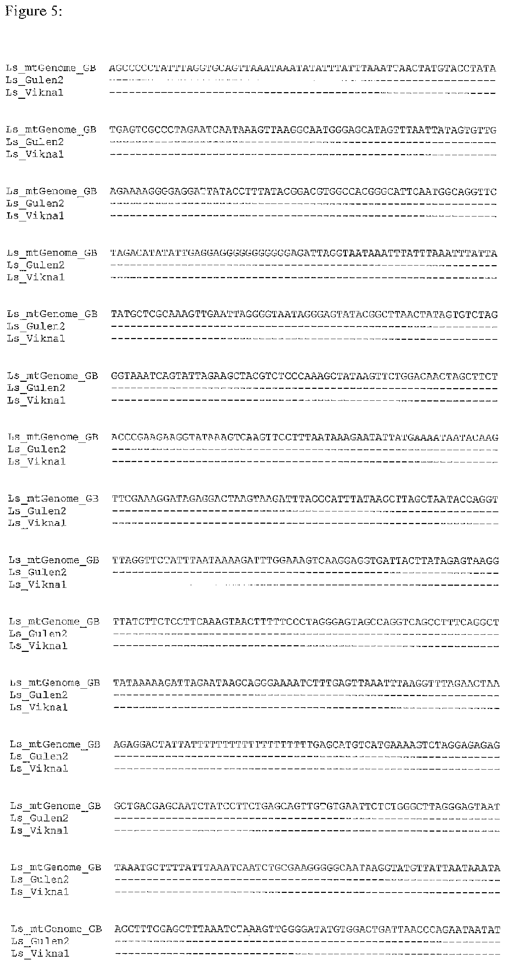

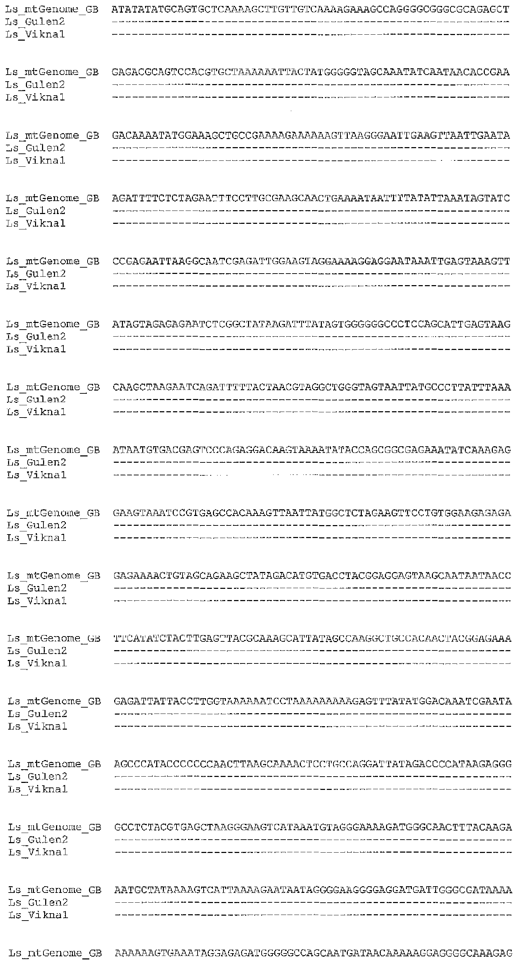

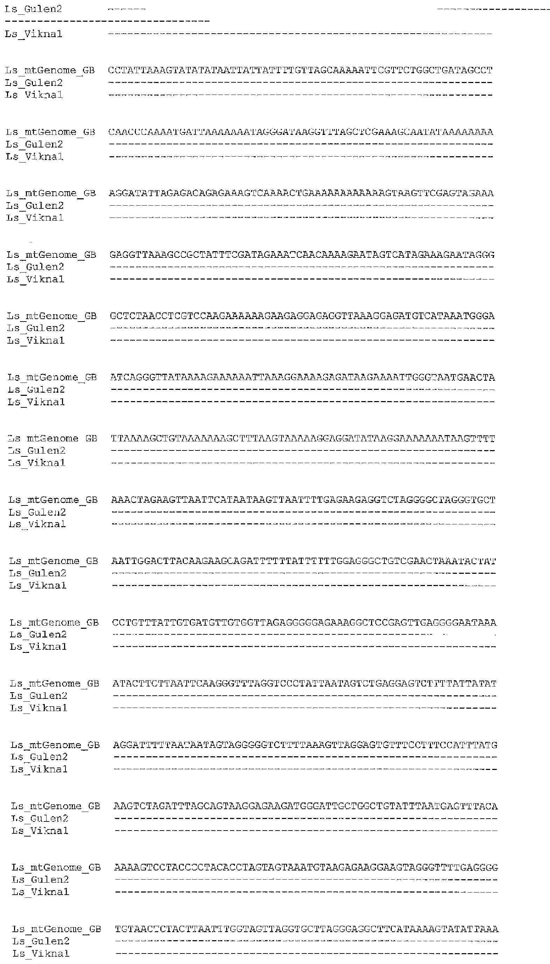

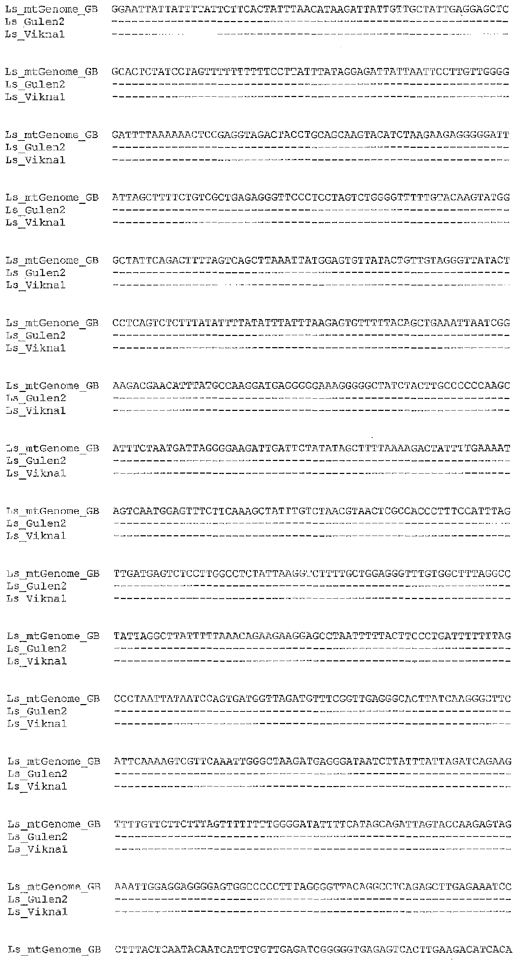

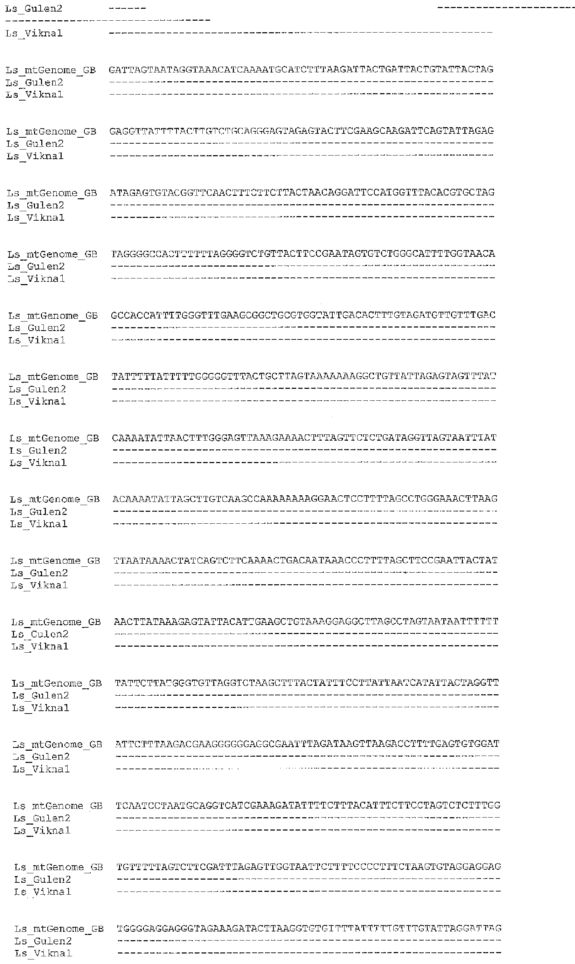

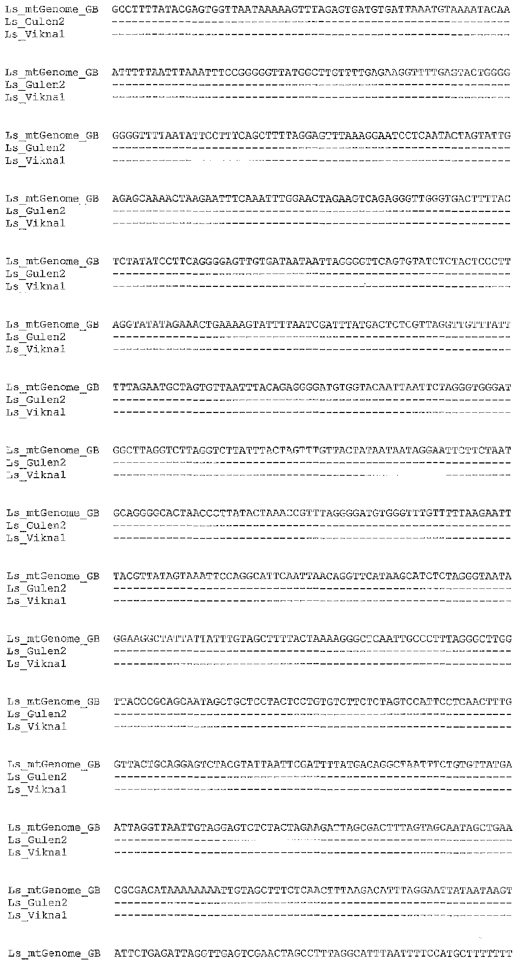

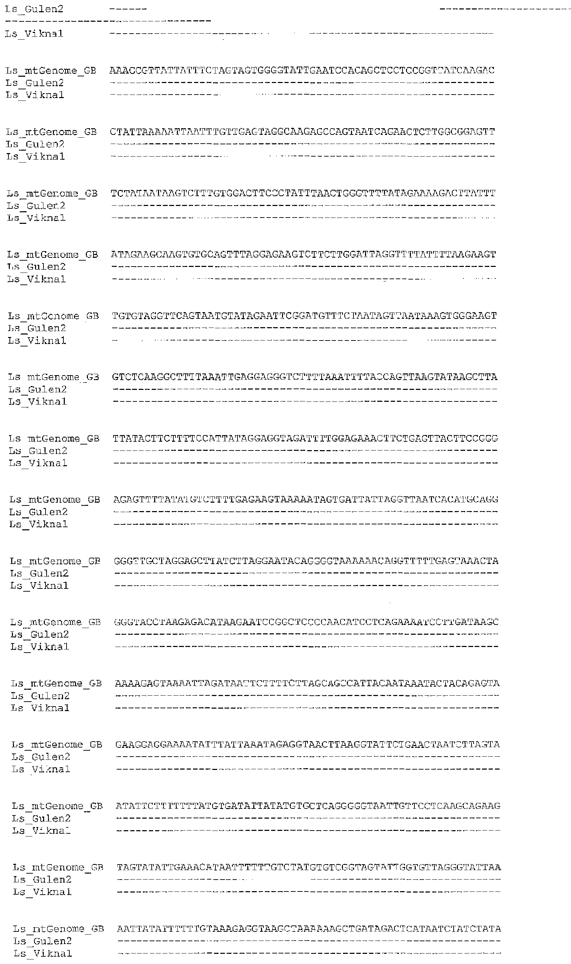

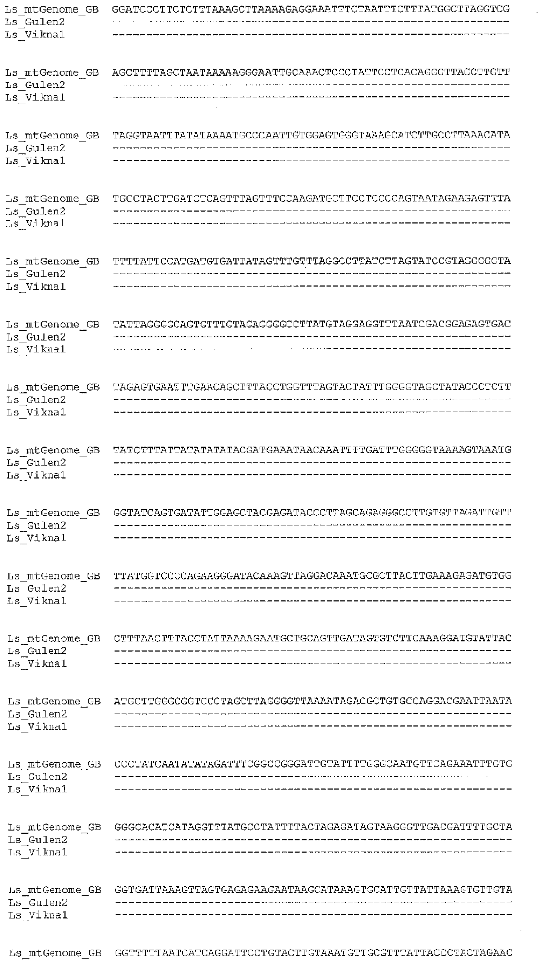

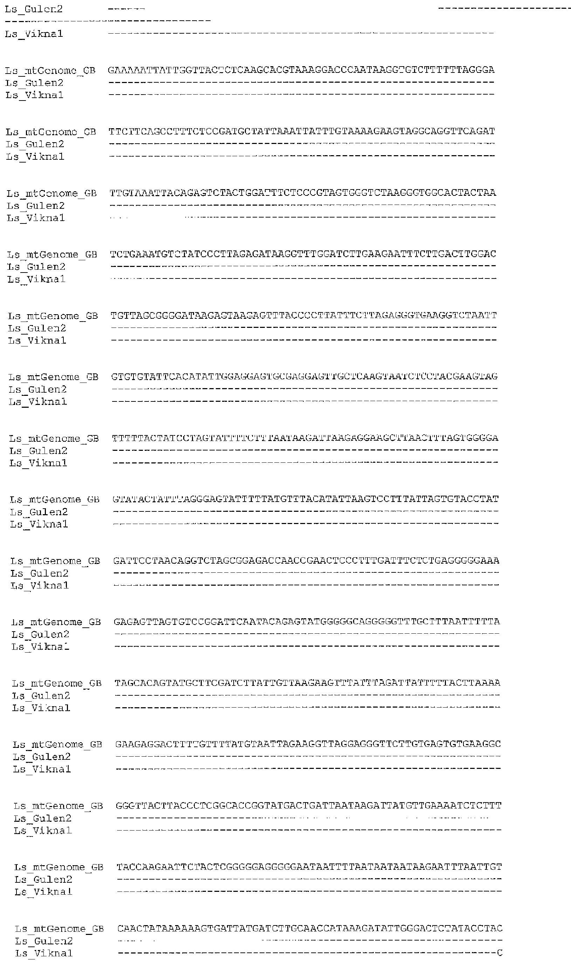

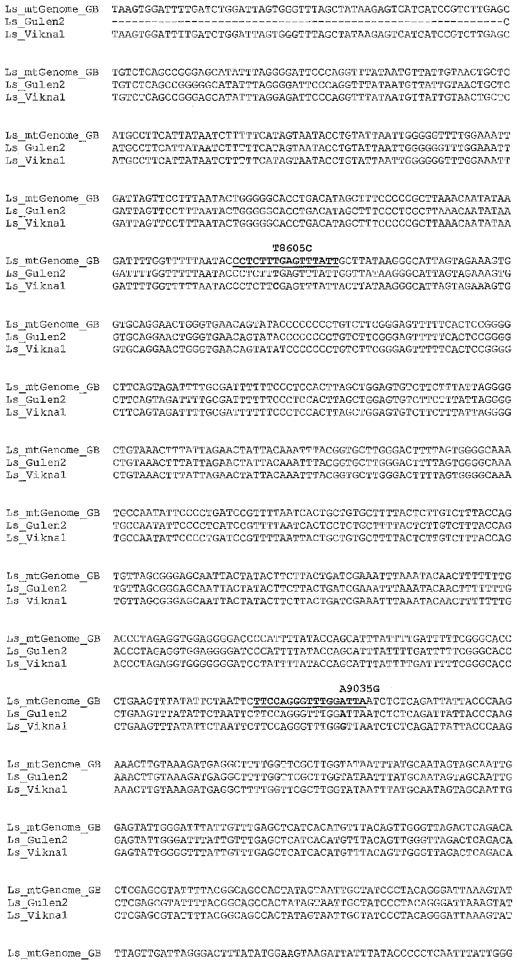

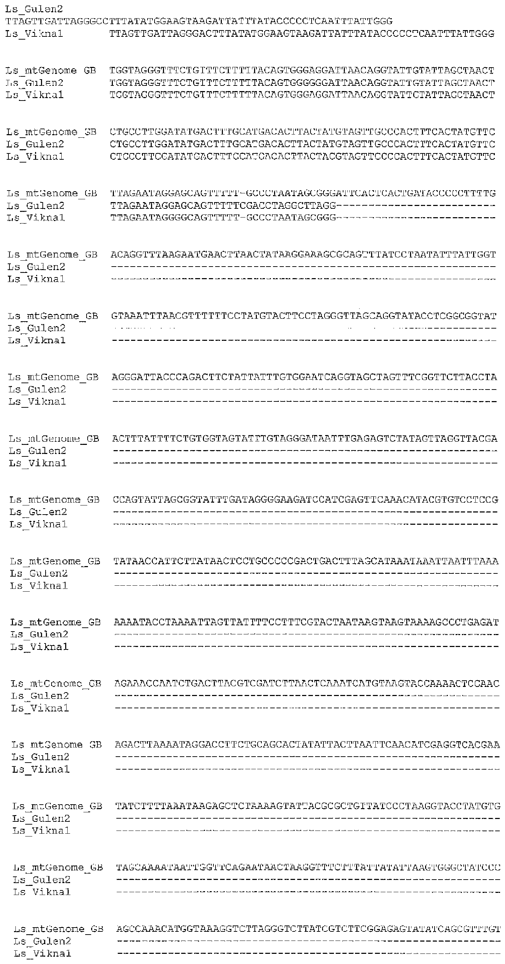

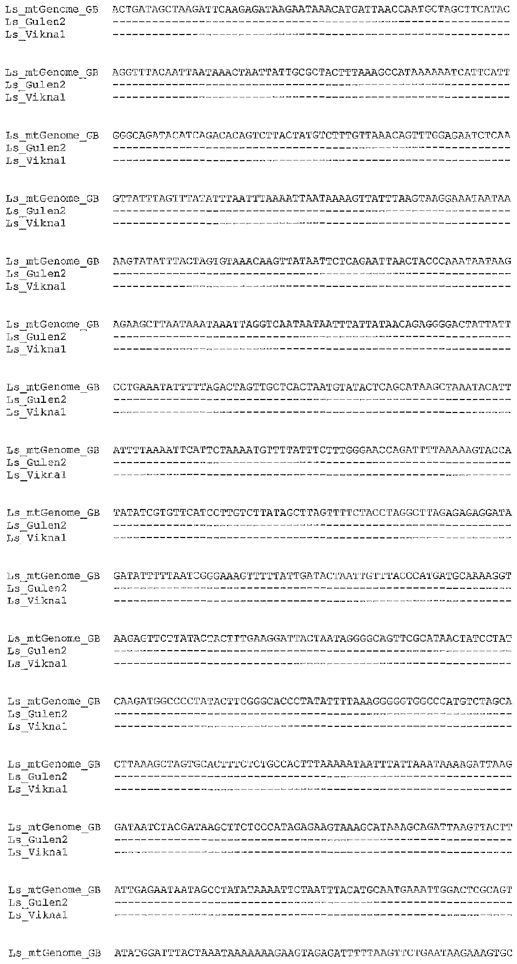

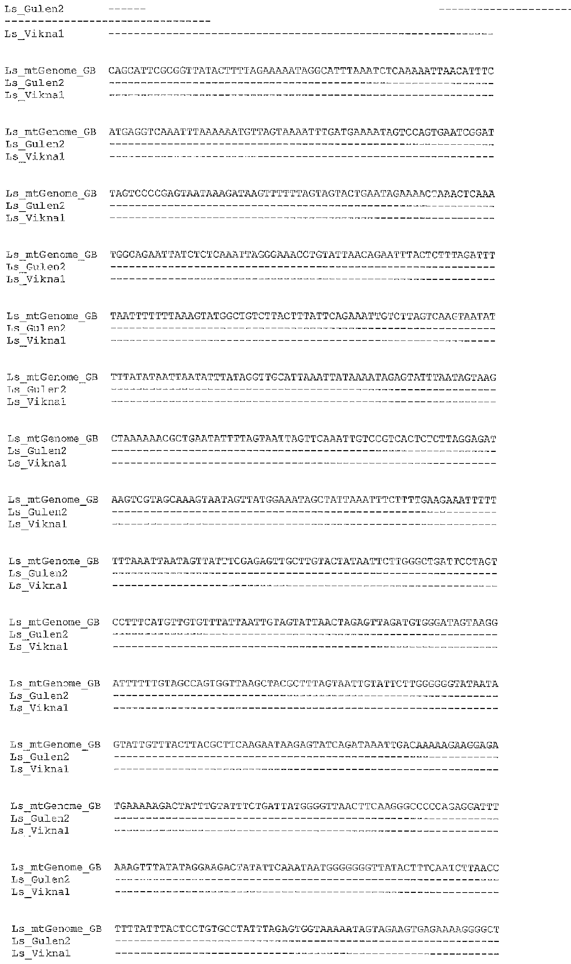

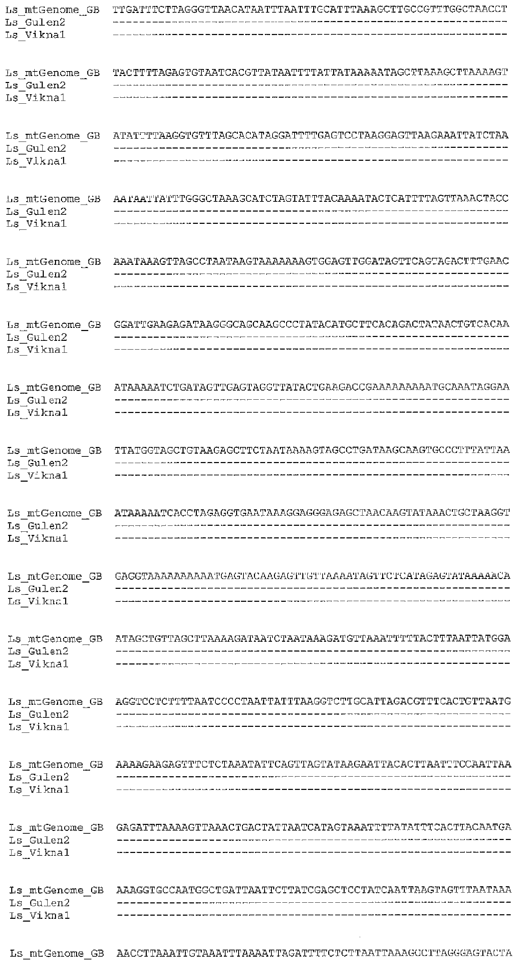

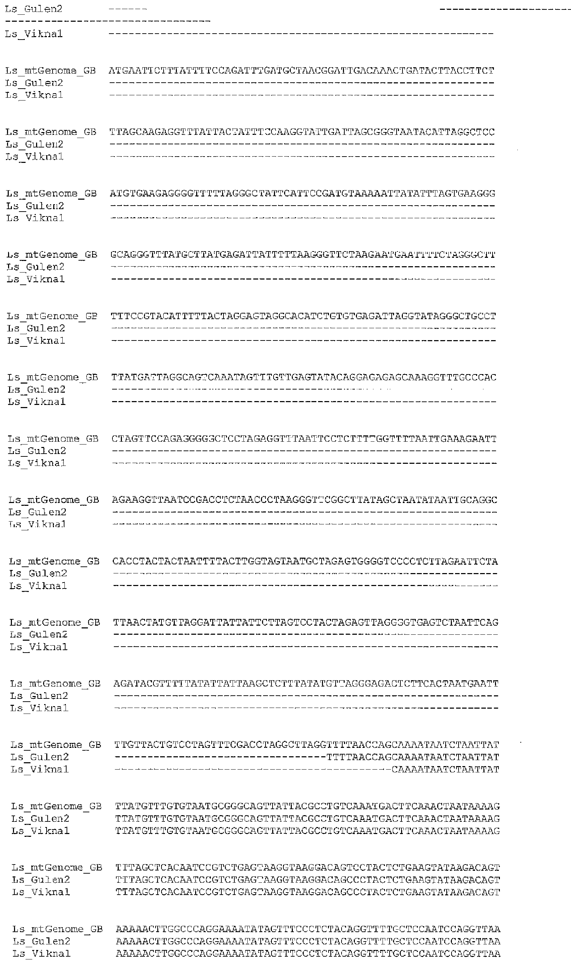

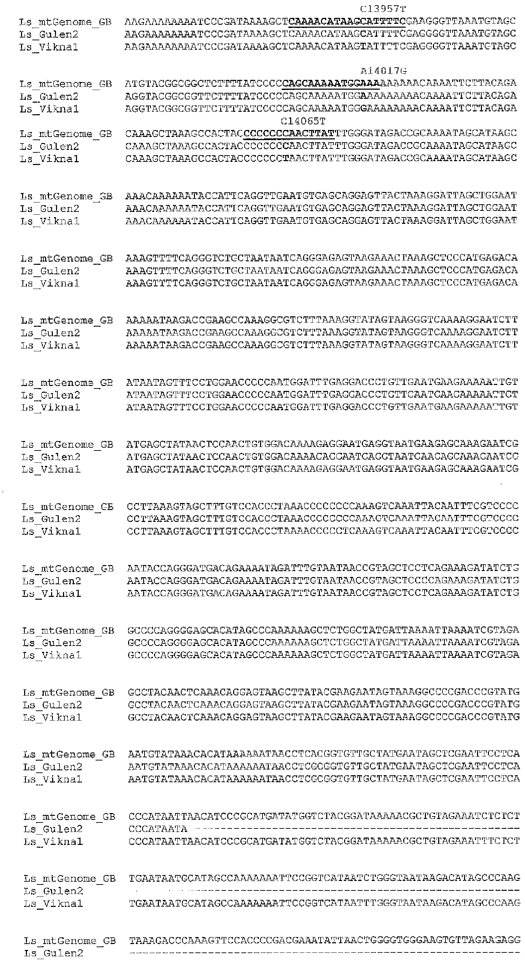

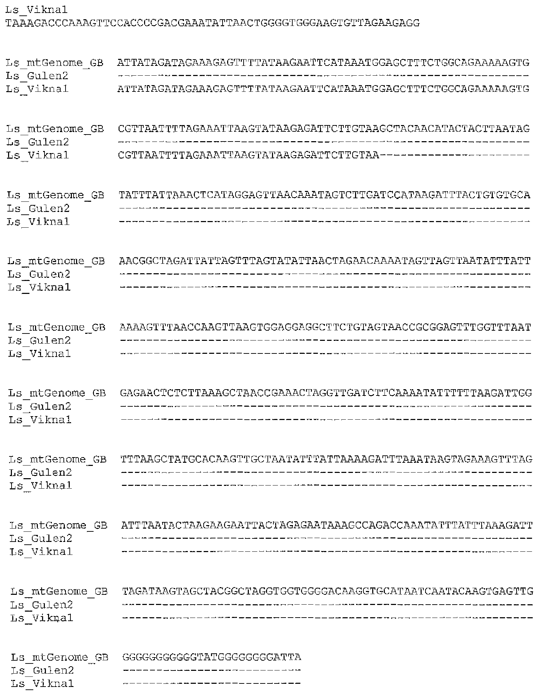

Throughout the application, the term "sea lice" is to be understood to mean any copepod belonging to the family Caligidae. However, whenever the term "sea lice" is used in connection with the experimental data in the present application, sea lice refers to the specie *Lepeophtheirus salmonis.* By hybridization of adult male sea lice shown to be sensitive to pyrethroids with preadult female sea lice shown to be resistant towards pyrethroids, the present inventors where able to show that the resistant female sea lice transmitted their pyrethroid resistant characteristics to their progenies (cf. figure 1, example 1). Resistant adult male sea lice did not transfer their pyrethroid resistant characteristics when crossed with sensitive females. The mechanism of action could therefore be linked to the mitochondrion of the sea louse. Based on further extensive sequence analysis of mitochondrial genomes of pyrethroid resistant sea lice, and comparison with the mitochondrial genomic sequence of a known reference strain (GenBank acc. No. NC_007215 (Tjensvoll et al., 2005, Gene, 353 (2), 218-230), SEQ ID No. 1) and mitochondrial sequences of a pyrethroid sensitive sea lice strain (SEQ ID No. 2 and 3), the present inventors have identified five single nucleotide polymorphism sites that are clearly linked to pyrethroid resistance (figure 5) in sea lice.

More particularly, sequences from 180 individual *L. salmonis* obtained from 6 different locations (see Tjensvoll et al 2006, *supra*) from CoI and cyt b were compared to sequences from pyrethroid sensitive and resistant lice and the newly identified SNPs characteristic for resistant strains are only present in the resistant populations. Two of the SNPs uniquely associated with resistance to pyrethroids are found in the CoI gene, more specifically in position 8605 and position 9035 (T8605C, A9035G). Three SNPs found to be uniquely associated with resistance to pyrethroids is found in the cyt b gene, more specifically in position 13957 (C13957T), 14017 (A14017G), and 14065 (C14065T).

The start point of the mitochondrial DNA sequence of SEQ ID No. 1 is in the D-loop (i.e. the non-coding region of the mtDNA). The numbering of the positions of the identified SNPs are based of the mitochondrial sequence of Tjensvoll et al, 2005, supra, deposited with the GenBank Acc. No. NC_007215 as depicted in SEQ ID No. 1. It is to be understood that whenever referring to the positions of the SNPs identified according to the present invention, the numbering is throughout the present description made according to the numbering of the reference strain sequence (GenBank Acc. No. NC_007215) if not otherwise stated.

Based on the identification of the five SNPs responsible for pyrethroid resistance in sea lice, a method for determination of pyrethroid resistance in crustaceans, such as copepods, in particular in sea lice, e.g. *Lepeophtheirus salmonis* PRLS have been provided. More particular, a method for detection of PRLS is provided comprising the steps of detecting single nucleotide polymorphism (SNP) associated with pyrethroid resistance in the mitochondrial DNA of a sea lice to be analyzed, wherein said sea lice is resistant to pyrethroids if at least one of the following nucleotides:
i. C in position 8605;
ii. G in position 9035;
iii. T in position 13957;
iv. G in position 14017;
v. T in position 14065;
or the complementary oligonucleotide thereof,
is present in the mitochondrial DNA sequence of the sea lice, and wherein the numbering of said positions is in accordance with the sequence depicted in SEQ ID No. 1.

In addition to the qualitative determination of the SNPs involved in pyrethroid resistance according to the present invention, the present invention also provides for a method for gradation of crustacean population being susceptible of developing resistance, i.e. taking into account the composition of haplotypes that are determined within a crustacean population (see example 3 for practical details).

According to the present invention, "single-nucleotide polymorphisms (SNP)" is to be understood to refer to a nucleotide sequence variation occurring when a single nucleotide, A, T (U), C or G in the genome, or other shared sequences (e.g. RNA) or fragments thereof, differs between members of a biological species, such as between variants of *Lepeophtheirus salmonis.* SNPs may fall within coding sequences of genes, or non-coding regions of genes, or in the regions between the genes in a genome. The five SNPs identified according to the present invention fall within the genes encoding the cytochrome oxidase subunit I (CoI) and cytochrome B (Cytb), respectively.

Furthermore, as used herein, an "oligonucleotide sequence" or "nucleic acid sequence" is generally an oligonucleotide sequence or a nucleic acid sequence containing a SNP described herein, or one that hybridizes to such molecule such as a nucleic acid sequence with a complementary sequence.

The skilled person is well aware of the fact that nucleic acid molecules may be double - stranded or single-stranded, and that reference to a particular site of one strand refers, as well, to the corresponding site on a complementary strand. Thus, when defining a SNP position, reference to an adenine (A), a thymine (T) (uridine (U)), a cytosine (C) or a guanine (G) at a particular site on one strand of a nucleic acid is also to be understood to define a thymine (uridine), adenine, guanine, or cytosine, respectively, at the corresponding site on a complementary strand of the nucleic acid molecule. Thus, reference may be made to either strand in order to refer to a particular SNP position. The oligonucleotide probes and oligonucleotide primers according to the present invention may be designed to hybridize to either strand, and SNP detection methods disclosed herein may thus also in general target either strand.

An "isolated nucleic acid" as used herein is generally one that contains at least one of the SNPs described herein or one that hybridizes to such molecule, e.g. a nucleic acid with a complementary sequence, and is separated from most other nucleic acids present in the natural source of the nucleic acid, and is thus substantially free of other cellular material.

### Oligonucleotide probes and oligonucleotide primers

The present invention provides oligonucleotide probes and oligonucleotide primers that may be used for detection of the presence of the SNPs according to the present invention in mitochondrial DNA of a sea lice to be tested, and thus for determination of pyrethroid resistance. The detection of nucleic acids present in a biological sample is widely applied in both human and veterinary diagnosis, wherein nucleic acids from e.g. pathogens present in biological samples are isolated and hybridized to one or more hybridizing probes or primers are used in order to amplify a target sequence.

One or more oligonucleotide probes may be constructed based on the teaching herein and used in hybridization based detection methods where upon the binding of the oligonucleotides to the target sequence enables detection of the presence of at least one of the SNPs described herein if present in the sample to be tested.

The skilled person will acknowledge that an oligonucleotide probe according to the present invention may be a fragment of DNA or RNA of variable length used herein in order to detect an SNP in a target sequence, e.g. single-stranded mitochondrial DNA or RNA, upon hybridization of the oligonucleotide probe to complementary sequence(s) of the said target sequence to be analyzed. The oligonucleotide probe according to the present invention may furthermore be labeled with a molecular marker in order to easily visualize that hybridization, and thus detection of the SNPs disclosed herein, have been achieved. Molecular markers commonly known to the skilled person may be used, e.g. a radiolabel, and more preferably, a luminescent molecule or a fluorescent molecule enabling the visualisation of the binding of the probe(s) to a target sequence.

A oligonucleotide probe according to the present invention is able to hybridize to another nucleic acid molecule, such as the single strand of mitochondrial DNA or RNA originating from a sea lice to be analysed, under appropriate conditions of temperature and solution ionic strength, cf. e.g. Sambrook et al., Molecular Cloning: A laboratory Manual (third edition), 2001, CSHL Press, (ISBN 978-087969577-4). The condition of temperature and ionic strength determine what the skilled person will recognise as the "stringency" of the hybridization. The suitable stringency for hybridisation of a probe to target nucleic acids depends on inter alia the length of the probe and the degree of complementation, variables well known to the skilled person. A oligonucleotide probe according to the present disclsoure typically comprises a nucleotide sequence which under stringent conditions hybridize to at least 8, 10, 12, 16, 20, 22, 25, 30, 40, 50 (or any other number in-between) or more consecutive nucleotides in a target nucleic acid molecule, e.g. single-stranded mitochondrial DNA or RNA isolated from the sea lice to be analyzed according to the present invention. According to one embodiment, the oligonucleotide probe according to the present disclsure comprises about 13 to 25 consecutive nucleotides. It is to be understood that the oligonucleotide probe according one embodiment comprise one of the SNPs described herein or the complement thereof. New technology like specific Locked Nucleic Acid (LNA) hybridization probes allows for the use of extremely short oligonucleotide probes (You Y.; Moreira B.G.; Behlke M.A. and Owczarzy R. (2006), "Design of LNA probes that improve mismatch discrimination, Nucleic Acids Res. 34 (8): e60) According to one embodiment, probes are provided which are selected from the group consisting of SEQ ID No. 16-20.

The present invention furthermore provides oligonucleotide primers useful for amplification of any given region of a nucleotide sequence, in particular a region containing one of the SNPs described herein. An oligonucleotide primer according to the present disclosure typically comprises a nucleotide sequence at least 8, 10, 12, 16, 20, 22, 25, 30, 40, 50 (or any other number in-between) or more consecutive nucleotides.

According to one embodiment, the oligonucleotide primer according to the present disclosure comprises about 18 - 25 consecutive nucleotides, more preferably about 20 nucleotides.

As used herein, the term "oligonucleotide primer" is to be understood to refer to a nucleic acid sequence suitable for directing an activity to a region of a nucleic acid, e.g. for amplification of a target nucleic acid sequence by polymerase chain reaction (PCR). According to one embodiment of the present invention, "oligonucleotide primer pairs" is provided suitable for amplification of a region of mitochondrial genome material comprising the SNPs according to the present invention.

The skilled person will acknowledge that an oligonucleotide primer according to the present invention may be a fragment of DNA or RNA of variable length used herein in order to detect an SNP in a target sequence, e.g. single-stranded mitochondrial DNA or RNA, upon alignment of the oligonucleotide probe to complementary sequence(s) of the said target sequence to be analyzed. An oligonucleotide primer according to the present invention may furthermore be labeled with a molecular marker in order to enable visualization of the results obtained. Various molecular markers or labels are available, dependent on the SNP detection method used.

An oligonucleotide primer according to the present invention typically comprises the appropriate number of nucleotides allowing that said primer align with the target sequence to be analyzed. It is to be understood that the oligonucleotide primer according to the present invention according to one embodiment comprises the SNP described herein or the complement thereof. According to one embodiment, the probes useful in order to determine pyrethroid resistant sea lice is selected from the group consisting of SEQ ID No. 16, SEQ ID No.17, SEQ ID No.18, SEQ ID No.19, and SEQ ID No.20.

According to one embodiment of the present invention, primer pairs are provided selected from the group consisting of SEQ ID No. 6 - SEQ ID No. 15 (see also table 5 below). Oligonucleotide probes and oligonucleotide primers according to the present invention may be synthesized according to methods well known to the skilled person.

The present discclosure furthermore relates to isolated nucleic acid sequences and variants or fragments thereof having at least 70% identity with the nucleic acid sequences depicted in SEQ ID NO. 4, or SEQ ID No. 5, or fragments thereof. The term "% identity" is to be understood to refer to the percentage of nucleotides that two or more sequences or fragments thereof contains, that are the same. A specified percentage of nucleotides can be referred to as e.g. 70% identity, 80% identity, 85% identity, 90% identity, 95% identity, 99% identity or more (or any number in between) over a specified region when compared and aligned for maximum correspondence. The skilled person will acknowledge that various means for comparing sequences are available. For example, one non-limiting example of a useful computer homology or identity program useful for determining the percent homology between sequences includes the Basic Local Alignment Search Tool (BLAST) (Altschul et al., 1990, J. of Molec. Biol., 215:403-410, "The BLAST Algorithm; Altschul et al., 1997, Nuc. Acids Res. 25:3389-3402, , Karlin and Altschul 1990, Proc. Nat'l Acad. Sci. USA, 87:2264-68; 1993, Proc. Nat'l Acad. Sci. USA 90:5873-77).

### SNP identification methods

Upon the identification of the SNPs associated with pyrethroid resistance in sea lice according to the present invention, the skilled person will acknowledge that various methods commonly used in order to detect polymorphisms within a population of sea lice may be used. Both methods based on genome sequencing, hybridization and enzyme based methods are applicable for determining whether a sea louse is pyrethroid resistant in accordance with the present inventions.

Various enzyme based methods are available for the skilled person, of which a number of polymerase chain reaction (PCR) based methods are available.

For example, Perkin Elmer Life Sciences provides SNP detection kit that may be used in order to determine whether a sea louse is pyrethroid resistant (AcycloPrime™-FP SNP Detection). In said method, a thermostable polymerase is used which extends an oligonucleotide primer according to the present invention by one base, then ending the oligonucleotide primer one nucleotide immediately upstream of the relevant SNP position by the incorporation of fluorescent dye-labeled terminators. The identity of the base added is then determined by the increase fluorescence polarization of its linked dye (http://shop.perkinelmer.com/Content/Manuals/MAN_AcycloPrimeSNPKit.pdf).

Oligonucleotide primers according to the present invention useful in such a method would thus be constructed in order to facilitate the extension of the primer by one base in the position selected from the group 8605, 9035, 13957, 14017, or 14064, relative to SEQ ID No. 1.

Another enzyme based method that may be used is restriction fragment length polymorphism (RLFP), utilizing that various, highly specific endonuclease upon digestion of the target sample, i.e. mitochondrial genome, results in different fragments that may be separated by gel electrophoresis.

Yet another enzyme based method that may be utilized in accordance with the present invention is the flap endonuclease (FEN) method. In said method, a structure-specific endonuclease is used to cleave a three-dimensional complex formed by hybridization with the target DNA, e.g. mitochondrial genomic material from sea lice, and where annealing with a target sequence comprising the SNP of interest triggers cleavage by the endonuclease (Oliver, 2005, Mutat. Res., vol. 573 (1-2), pp. 103-110).

Yet another method applicable in respect of the present invention is based on the use of TaqMan® Assays (Invitrogen). In said assay the oligonucleotide primers used in order to detect an SNP is labeled in both the 5'- and the 3' end, i.e. with a fluorophore at the 5'end of the oligonucleotide primer, and a quencher at the 3'-end of the oligonucleotide primer. Upon annealing of the oligonucleotide primer with a target sequence, the Taq polymerase will extend the oligonucleotide primer and form a nascent strand, followed by degradation of the oligonucleotide primer being annealed to the target, said degradation eventually resulting in the release of the fluorophore and provide a cleavage close to the quencher. The fluorescence signal produced is proportional to the fluorophore released. Various fluorophore labels may be used, such as e.g. 6-carboxyfluorescein, tetrafluorofluorescein. As quenchers, tetramethylrhodamine or dihydrocyclopyrroloindol may be used.

Several hybridization methods for detection of SNPs are available to the skilled person, and which may be utilized in accordance with the method of the present invention. For example, the SNPs according to the present invention may be detected utilizing molecular beacon technology. According to this aspect of the present invention, oligonucleotide primers may be synthesized comprising complementary regions at each end allowing the formation of a hairpin loop, and wherein a fluorophore is attached at one end of the oligonucleotide primer, and a quenching agent is attached to the other end, and wherein fluorescence signal is produced upon binding to a DNA target of interest, i.e. mitochondrial genomic material isolated from the sea louse to be analyzed.

Yet another method applicable in respect of the present invention is based on DNA or RNA sequencing, which is the process of determining the precise order of nucleotides within a molecule. It includes any method or technology that is used to determine the order of the four bases (adenine, guanine, cytosine, and thymine) in a strand of DNA. The skilled person is well known with the various commonly known DNA and RNA sequencing methods that may be used according to the present invention, such as e.g. shotgun sequencing or bridge PCR sequencing.

### Isolation of sea lice genomic material

The method according to the present invention may according to one embodiment involve in an isolated biological sample from a sea lice testing for the presence of a SNP associated with PRLS in the mitochondrial genome. The skilled person will acknowledge that the SNPs identified according to the present invention may be detected by analyzing mitochondrial DNA as well as mitochondrial RNA, dependent upon the detection method used.

In order to determine whether a sea louse is pyrethroid resistant in accordance with the present invention, mitochondrial genomic material may be isolated. Various methods for obtaining genomic material well known to the skilled person are available. The skilled person will acknowledge that any tissue (i.e. any part of the sea lice) may be used in order to extract mitochondrial genomic material. Furthermore, the mitochondrial genomic material to be analyzed according to the present invention may be obtained from sea lice of any life stages, e.g. the free swimming stages (nauplius stage I and II), the copepod stage, the pre-adult (chalimus stages 1-4), or the adult stage (adult male or adult female). According to one embodiment, tissue removed from sea lice to be tested is maintained in 70% ethanol or other conservation liquid prior to further isolation of genomic material. DNA may be extracted from the obtained tissue using commonly available DNA extraction/isolation methods, such as e.g. DNeasy DNA Tissue Kit according to the protocol of the manufacturer (http://lycofs01.lycoming.edu/∼gcat-seek/protocols/DNeasy_Blood_&_Tissue_Handbook.pdf)

Still another method applicable for detecting SNP is High Resolution Melting Analysis (HRM) enabling rapid detection of SNPs and determination of genetic variation within a population. The first step of a HRM protocol consist often of amplification of the region of interest, using standard nucleotide sequence amplification techniques well known to the skilled person, and wherein the amplification is performed in the presence of a specialized double-stranded DNA binding dye being highly fluorescent when bound to dsDNA and poorly fluorescent in unbound state. This difference provides for the monitoring of the DNA amplification. After amplification, the target is gradually denatured by increasing the temperature in small increments, resulting in a characteristic melting profile. As the amplified DNA is denatured gradually, dye is released, thus resulting in a drop in fluorescence.

### SNP detection Kits

Based on the teaching herein, the skilled person will acknowledge that, based on the identified SNPs and associated sequence information disclosed herein, detection reagents can be developed and used to determine any SNP described herein individually or in combination, and that such detection reagents can be readily incorporated into kits used for SNP detection known in the art. The term "kit" as used herein in the context of SNP detection reagents are intended to cover e.g. combinations of multiple SNP detection reagents, or one or more SNP detection reagents, such as oligonucleotide probe(s) and oligonucleotide primer(s) or primer sets, arrays/microarrays of nucleic acid molecules, and beads that contain one or more oligonucleotide probe(s), oligonucleotide primer(s) or other detection reagents useful in the method of the present invention. It is furthermore to be understood that the SNP detection reagents in a kit according to the present invention may furthermore include other components commonly included in such kits, e.g. such as various types of biochemical reagents (buffers, DNA polymerase, ligase, deoxynucleotide triphosphates for chain extension/amplification, etc.), containers, packages, substrates to which SNP detection reagents are attached., etc. necessary to carry the method according to the present invention. According to one embodiment of the present invention, a kit is provided which comprises the necessary reagents to carry out one or more assays in order to detect the SNP disclosed herein according to the method of the present invention. A kit according to the present invention may preferably comprise one or more oligonucleotide probes that hybridize to a nucleic acid target molecule (i.e. mitochondrial genetic material) enabling detection of each target SNP position if present in the material analyzed. Multiple pairs of probes may be included in the kit to simultaneously analyze for the presence of the SNP disclosed herein at the same time. The probes contained in the kit according to the present invention may according to one embodiment be immobilized on a carrier, such as e.g. an array or a bead.

According to one embodiment, the oligonucleotide probes are suitable for the detection of the SNP T8605C. According to another embodiment of the disclosure, the oligonucleotide probes are suitable for the detection of the SNP A9035G. According to yet another embodiment, the oligonucleotide probes is suitable for the detection of the SNP C13957T. According to yet another embodiment, the oligonucleotide probes is suitable for the detection of the SNP A14017G. According to yet another embodiment, the oligonucleotide probes is suitable for the detection of the SNP C14065T. According to yet another embodiment, the kit according to the present disclosure comprises oligonucleotide probes suitable for detection of all the SNPs described herein.

According to one embodiment, a kit according to the present invention comprises oligonucleotide primer(s) and optionally further SNP detection reagents useful in SNP detection methods utilizing oligonucleotide primers or primer pair(s). According to one embodiment, the kit according to the present disclosure comprises a forward primer and a reverse primer for amplifying a region containing a SNP selected from SNP selected from the group of SNPs consisting of T8605C, (U8605C in case of RNA), A9035G, C13957T (or U13957T in case of RNA), A14017G, or C14065T (C14065U in case of RNA). Said kit may furthermore optionally comprise further SNP detection reagents (enzymes and nucleotide triphosphates) necessary for conducting PCR or real time PCR. According to one embodiment, the primer pairs are suitable for the detection of the SNP T8605C. According to another embodiment, the primer pairs are suitable for the detection of the SNP A9035G. According to yet another embodiment, the primer pairs is suitable for the detection of the SNP C13957T. According to yet another embodiment, the primer pairs is suitable for the detection of the SNP A14017G. According to yet another embodiment, the primer pairs is suitable for the detection of the SNP C14065T. According to yet another embodiment, the kit according to the present invention comprises primer pairs suitable for detection of all the SNPs described herein.

**Table 1 Sequences related to the invention**

| SEQ ID No. | Description of sequence |
|---|---|
| 1 | Mitochondrial DNA of Lepeophtheirus salmonis from sea lice (GenBank acc. No. NC_007215 (Tjensvoll et al., 2005, Gene, 353 (2), 218-230) |
| 2 | Mitochondrial DNA of Lepeophtheirus salmonis isolated from pyrethroid sensitive strain (Ls_Gulen2 CoI) |
| 3 | Mitochondrial DNA of Lepeophtheirus salmonis isolated from pyrethroid sensitive strain (Ls_Gulen2 Cytb) |
| 4 | Mitochondrial DNA of Lepeophtheirus salmonis isolated from pyrethroid resistant strain (Ls_Vikna1 CoI) |
| 5 | Mitochondrial DNA of Lepeophtheirus salmonis isolated from pyrethroid resistant strain (Ls_Vikna1 Cytb) |
| 6 | Oligonucleotide (forward) primer useful for detecting SNP T8605C |
| 7 | Oligonucleotide (reverse) primer useful for detecting SNP T8605C |
| 8 | Oligonucleotide (forward) primer useful for detecting SNP A9035G |
| 9 | Oligonucleotide (reverse) primer useful for detecting SNP A9035G |
| 10 | Oligonucleotide (forward) primer useful for detecting SNP C13957T |
| 11 | Oligonucleotide (reverse) primer useful for detecting SNP C13957T |
| 12 | Oligonucleotide (forward) primer useful for detecting SNP A14017G |
| 13 | Oligonucleotide (reverse) primer useful for detecting SNP A14017G |
| 14 | Oligonucleotide (forward) primer useful for detecting SNPC14065T |
| 15 | Oligonucleotide (reverse) primer useful for detecting SNP C14065T |
| 16 | Oligonucleotide probe useful for detecting SNP T8605C |
| 17 | Oligonucleotide probe useful for detecting SNP A9035G |
| 18 | Oligonucleotide probe useful for detecting SNP C13957T |
| 19 | Oligonucleotide probe useful for detecting SNP A14017G |
| 20 | Oligonucleotide probe useful for detecting SNP T8605C |

According to one embodiment of the present invention, a method is provided for detection of the pyrethroids deltamethrin (CAS No. 52918-63-5) and cis-cypermethrin (CAS No. 52315-07-8). It is well known that resistance against one type of pyrethroid in general provides the possibility of resistance also against all type of pyrethroids; see e.g. Sevatdal S, Fallang A, Ingebrigtsen K, Horsberg TE. 2005. Monooxygenase mediated pyrethroid detoxification in sea lice (Lepeophtheirus salmonis). Pest Manag Sci. 2005 Aug;61(8):772-8, Du Y, Nomura Y, Satar G, Hu Z, Nauen R, He SY, Zhorov BS, Dong K. 2013. Molecular evidence for dual pyrethroid-receptor sites on a mosquito sodium channel. Proc Natl Acad Sci USA. 2013 Jul 2. [Epub ahead of print], and Zhu F, Gujar H, Gordon JR, Haynes KF, Potter MF, Palli SR. 2013. Bed bugs evolved unique adaptive strategy to resist pyrethroid insecticides. Sci Rep. 2013; 3:1456. doi: 10.1038/srep01456.

The skilled person will thus acknowledge, based on the teaching of the present invention, that the method according to the invention may be used to determine resistance towards various types of pyrethroids in crustaceans, such as e.g. allethrin, bifenthrin, cyfluthrin, cyphenothrin, esfenvalerat, etofenoprox, fenpropathrin, fenvalerate, flumethrin, flucythrinate, imiprothrin, lamda-cyhalothrin, metofluthrin, permethrin, prallethrin, resmethrin, silafluofen, sumithrin, fluvalinate, teflutrhin, and tetramethrin.

### Examples

### Example 1: Hybridization of sensitive and pyrethroid resistant sea lice

### Crossing experiment

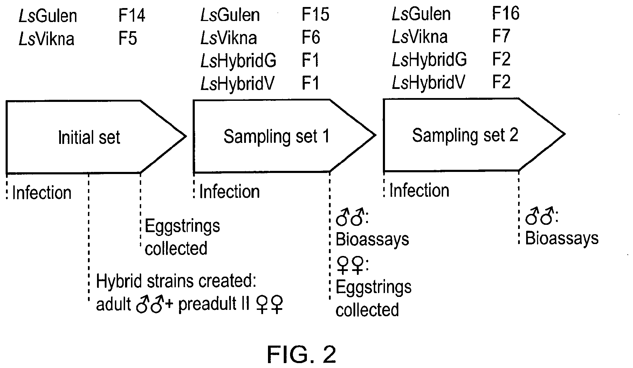

In order to investigate the inheritance and mechanism of pyrethroid resistance in *L*. *salmonis,* two strains (sensitive and resistant) and their reciprocal hybrids were used for a two-generation breeding experiment complimented with bioassays to measure tolerance (Fig. 2). The pyrethroid sensitive strain (hereon also referred to as *Ls*Gulen) was collected in June 2006 from newly slaughtered rainbow trout from Gulen in western Norway. The pyrethroid resistant strain (hereon also referred to as *Ls*Vikna) was collected in February 2009 from a salmon farm in Vikna, North Trøndelag, Norway. The resistant strain was collected on the basis of suspected reduced sensitivity to pyrethroids due to the fact that there had been reported a treatment failure on the farm from which it was collected. This was subsequently confirmed in bioassays with *Ls*Gulen as sensitive control.

Prior to the initiation of the present study, *Ls*Gulen and *Ls*Vikna had been cultivated in laboratory for 13 and 4 generations respectively under standard rearing conditions at the Institute of Marine Research (IMR), Norway (Hamre et al., 2009, Establishment and characterisation of salmon louse (Lepeophtheirus salmonis (Krøyer 1837)) laboratory strains. Parasitology International, 58, 451-460).

In order to hybridise the lice strains for the inheritance experiment, the parental strains were first synchronised and multiplied up in numbers by simultaneously infecting 20 Atlantic salmon with copepods. In total, 1000 copepods per strain were added. At 32 days post infection (dpi), all salmon were anaesthetised and approximately half of the lice were carefully removed using tweezers. At this stage, lice were predominantly preadult 2 females and adult males, with a few percent adult females and preadult 2 males. The lice removed from the salmon were sexed, and then used to create the reciprocal hybrid strains. This was achieved by combining 4-5 females from the resistant strain and 4-5 males from the sensitive strain (*Ls*HybridV), and 4-5 females from the sensitive strain and 4-5 males from the resistant strain (*Ls*HybridG). Only preadult 2 females were used. These combinations were each placed into 2 replicate tanks in order to make two hybrid strains (4 groups and 8 tanks at this stage). At 67-68 dpi, all lice were removed from the four strains. Egg strings were removed from adult females and incubated in containers containing a single strain each. The number of adult lice, and harvested egg strings varied, but all were incubated (Table 2).

**Table 2. Number of egg strings collected from each strain. Egg strings from replicate tanks were pooled. Most egg strings were part of a pair.**

| Strain | Generation | Egg strings (n) |
|---|---|---|
| *Ls*Gulen | F14 | 133 |
| *Ls*Vikna | F5 | 106 |
| *Ls*HybridG | P | 96 |
| *Ls*HybridV | P | 60 |

The subsequent generation (F2, Fig. 2) were both produced using the general procedure described above, although the hybrid strains were only crossed back to themselves to create multiple generation-hybrids. Collection of egg strings, which marked the establishment of a new generation, was done at 55 and 56 dpi respectively. Salmon that hosted the previous generation were re-used for reinfection with the next generation, but hosted only the same experimental strain.

### Bioassay test

Each strain, including both types of hybrids obtained in example 1 were quantified in their tolerance of deltamethrin at sampling points 1 and 2 representing the F1 and F2 generations for the hybrids (Fig. 1 and 2). Bioassays were performed according to standard guidelines (SEARCH Consortium, 2006, Sea lice resistance to chemotherapeutants: A handbook in resistance management. 2 ed.), with minor modifications. Only adult male lice were used for the bioassay, which after sampling (at 55 or 56 dpi) had been incubated overnight in running seawater (ca. 10 °C). The 30 min exposure to the prepared deltamethrin concentration was also carried out at ca. 10 °C, and higher concentrations than those described in the given protocol were included (Table S3). After exposure to deltamethrin, the lice were kept in running seawater (24 h at ca. 10 °C) until evaluation of the bioassays. At evaluation, lice were characterised as active, or inactive (moribund and dead lice pooled). EC₅₀ values were calculated using probit analysis (PoloPlus 2.0; LeOra Software, CA, USA). The results of two deltamethrin bioassay sets are shown in table 3a and 3b below.

**Table 3a. Deltamethrin bioassays performed on sampling set 1**

| | | | |
|---|---|---|---|
| Lice: | LsHybridG | | |
| Generation: | F1 | | |
| Fish tank: | 01 | | |

| Dose (ppb) | Active (n) | Inactive (n) | % Inactive |
|---|---|---|---|
| 0 | 17 | 0 | 0 |
| 0.1 | 16 | 0 | 0 |
| 0.3 | 32 | 9 | 21 |
| 1 | 0 | 35 | 100 |
| 3 | 0 | 28 | 100 |
| 6 | 0 | 18 | 100 |
| 12 | 0 | 27 | 100 |
| Lice: | LsHybridV | | |
| Generation: | F1 | | |
| Fish tank: | 02 | | |

| Dose (ppb) | Active (n) | Inactive (n) | % Inactive |
|---|---|---|---|
| 0 | 16 | 0 | 0 |
| 0.1 | 17 | 0 | 0 |
| 0.3 | 44 | 0 | 0 |
| 1 | 56 | 3 | 5 |
| 3 | 33 | 5 | 13 |
| 6 | 17 | 1 | 5 |
| 12 | 15 | 7 | 31 |
| Lice: | LsGulen | | |
| Generation: | F15 | | |
| Fish tank: | 04 | | |

| Dose (ppb) | Active (n) | Inactive (n) | % Inactive |
|---|---|---|---|
| 0 | 9 | 0 | 0 |
| 0.1 | 40 | 0 | 0 |
| 0.3 | 29 | 27 | 48 |
| 1 | 0 | 37 | 100 |
| 3 | 0 | 20 | 100 |
| 6 | 0 | 14 | 100 |
| 12 | 0 | 9 | 100 |
| Lice: | LsVikna | | |
| Generation: | F6 | | |
| Fish tank: | 05 | | |

| Dose (ppb) | Active (n) | Inactive (n) | % Inactive |
|---|---|---|---|
| 0 | 12 | 0 | 0 |
| 0.1 | 16 | 0 | 0 |
| 0.3 | 9 | 0 | 0 |
| 1 | 21 | 3 | 12 |
| 3 | 29 | 11 | 27 |
| 6 | 18 | 10 | 35 |
| 12 | 7 | 5 | 41 |
| Lice: | LsHybridV | | |
| Generation: | F1 | | |
| Fish tank: | 06 | | |

| Dose (ppb) | Active (n) | Inactive (n) | % Inactive |
|---|---|---|---|
| 0 | 26 | 0 | 0 |
| 0.1 | 17 | 0 | 0 |
| 0.3 | 45 | 0 | 0 |
| 1 | 39 | 3 | 7 |
| 3 | 36 | 1 | 2 |
| 6 | 19 | 2 | 9 |
| 12 | 19 | 9 | 32 |
| Lice: | LsHybridG | | |
| Generation: | F1 | | |
| Fish tank: | 07 | | |

| Dose (ppb) | Active (n) | Inactive (n) | % Inactive |
|---|---|---|---|
| 0 | 14 | 0 | 0 |
| 0.1 | 18 | 4 | 18 |
| 0.3 | 21 | 10 | 32 |
| 1 | 0 | 38 | 100 |
| 3 | 0 | 46 | 100 |
| 6 | 0 | 21 | 100 |
| 12 | 0 | 20 | 100 |
| Lice: | LsVikna | | |
| Generation: | F6 | | |
| Fish tank: | 09 | | |

| Dose (ppb) | Active (n) | Inactive (n) | % Inactive |
|---|---|---|---|
| 0 | 16 | 0 | 0 |
| 0.1 | 21 | 1 | 4 |
| 0.3 | 15 | 0 | 0 |
| 1 | 26 | 3 | 10 |
| 3 | 17 | 4 | 19 |
| 6 | 23 | 7 | 23 |
| 12 | 5 | 2 | 28 |
| Lice: | LsGulen | | |
| Generation: | F15 | | |
| Fish tank: | 10 | | |

| Dose (ppb) | Active (n) | Inactive (n) | % Inactive |
|---|---|---|---|
| 0 | 13 | 0 | 0 |
| 0.1 | 26 | 0 | 0 |
| 0.3 | 10 | 10 | 50 |
| 1 | 0 | 41 | 100 |
| 3 | 0 | 9 | 100 |
| 6 | 0 | 17 | 100 |
| 12 | 0 | 22 | 100 |

**Table 3b. Deltamethrin bioassays performed on sampling set 2.**

| | | | |
|---|---|---|---|
| Lice: | LsGulen | | |
| Generation: | F16 | | |
| Fish tank: | 03 | | |

| Dose (ppb) | Active (n) | Inactive (n) | % Inactive |
|---|---|---|---|
| 0 | 19 | 0 | 0 |
| 0.1 | 16 | 1 | 5 |
| 0.3 | 28 | 26 | 48 |
| 0.5 | 7 | 43 | 86 |
| 1 | 0 | 18 | 100 |
| 3 | 0 | 23 | 100 |
| 6 | 0 | 7 | 100 |
| Lice: | LsGulen | | |
| Generation: | F16 | | |
| Fish tank: | 04 | | |

| Dose (ppb) | Active (n) | Inactive (n) | % Inactive |
|---|---|---|---|
| 0 | 24 | 0 | 0 |
| 0.1 | 23 | 2 | 8 |
| 0.3 | 16 | 20 | 55 |
| 0.5 | 1 | 20 | 95 |
| 1 | 0 | 16 | 100 |
| 3 | 0 | 15 | 100 |
| 6 | 0 | 6 | 100 |
| 12 | 0 | 3 | 100 |
| Lice: | LsHybridV | | |
| Generation: | F2 | | |
| Fish tank: | 06 | | |

| Dose (ppb) | Active (n) | Inactive (n) | % Inactive |
|---|---|---|---|
| 0 | 19 | 0 | 0 |
| 0.1 | 13 | 0 | 0 |
| 0.3 | 14 | 0 | 0 |
| 0.5 | 15 | 0 | 0 |
| 1 | 32 | 0 | 0 |
| 3 | 25 | 5 | 16 |
| 6 | 22 | 1 | 4 |
| 12 | 11 | 3 | 21 |
| 20 | 6 | 4 | 40 |
| Lice: | LsHybridG | | |
| Generation: | F2 | | |
| Fish tank: | 07 | | |

| Dose (ppb) | Active (n) | Inactive (n) | % Inactive |
|---|---|---|---|
| 0 | 22 | 0 | 0 |
| 0.1 | 17 | 0 | 0 |
| 0.3 | 14 | 14 | 50 |
| 0.5 | 1 | 6 | 85 |
| 1 | 1 | 10 | 90 |
| 3 | 0 | 18 | 100 |
| 6 | 0 | 32 | 100 |
| 12 | 0 | 27 | 100 |
| 20 | 0 | 32 | 100 |
| Lice: | LsVikna | | |
| Generation: | F7 | | |
| Fish tank: | 08 | | |
| Dose (ppb) | Active (n) | Inactive (n) | % Inactive |
| 0 | 35 | 0 | 0 |
| 0.1 | 9 | 0 | 0 |
| 0.3 | 27 | 0 | 0 |
| 0.5 | 6 | 0 | 0 |
| 1 | 12 | 0 | 0 |
| 3 | 14 | 1 | 6 |
| 6 | 22 | 5 | 18 |
| 12 | 6 | 0 | 0 |
| 20 | 17 | 11 | 39 |
| Lice: | LsHybridV | | |
| Generation: | F2 | | |
| Fish tank: | 09 | | |
| Dose (ppb) | Active (n) | Inactive (n) | % Inactive |
| 0 | 16 | 0 | 0 |
| 0.1 | 12 | 2 | 14 |
| 0.3 | 11 | 0 | 0 |
| 0.5 | 28 | 1 | 3 |
| 1 | 17 | 1 | 5 |
| 3 | 17 | 0 | 0 |
| 6 | 25 | 6 | 19 |
| 12 | 11 | 1 | 8 |
| 20 | 14 | 17 | 54 |
| Lice: | LsHybridG | | |
| Generation: | F2 | | |
| Fish tank: | 10 | | |
| Dose (ppb) | Active (n) | Inactive (n) | % Inactive |
| 0 | 10 | 0 | 0 |
| 0.1 | 19 | 1 | 5 |
| 0.3 | 25 | 19 | 43 |
| 0.5 | 6 | 16 | 72 |
| 1 | 0 | 10 | 100 |
| 3 | 0 | 23 | 100 |
| 6 | 0 | 19 | 100 |
| 12 | 0 | 25 | 100 |
| 20 | 0 | 10 | 100 |

The four experimental strains were successfully cultured through the two generations and exposed to two separate bioassays. At the control and lowest concentration (0.1ppb), no differences in lice activity was observed between the four strains. This was the case for both sampling 1 and 2. However, at concentrations between 0.3 and 1 ppb, clear differences were observed between the strains (Fig. 2). Above 1 ppb, only lice from the resistant strain and its maternal hybrid strain survived the bioassay, while the sensitive strain and its associated maternal hybrid strain displayed 100% mortality. No differences between the hybrid strain and its maternal founder strain were observed for either strain, demonstrating that the resistance followed a maternal pattern of inheritance.

### Exemple 2: Identification of SNPs associated with pyrethroid resistance

The presented outcome of crossing resistant and sensitive lice (example 1) showed that the mitochondria genome encodes the resistance. The L. salmonis mtDNA was characterized by Tjensvoll et al (2005) and encodes 13 proteins, two rRNAs and 22 tRNA genes.

The first approach to identify genetic differences between the LsSensitive (LsGulen) and Ls Resistent (LsVikna) was by sequencing a 1000 bp fragment from *cytB* from 6 *L. salmonis* specimens (3 sensitive and 3 resistant). The sequencing revealed 3 unique SNPs in the *cytB* from the LsResistent strain. We sequenced the *cytB* from 10 more individual from the LsResistent strain, which revealed that all except 1 individual had the same unique SNP (C14065T) in the *cytB.* This single atypical LsVikna individual was sampled from the control group during the bioassay (i.e. LsVikna incubated without deltamethrin). Tjensvoll et al (2006) sequenced *cytB, A6. coI* and *16S* from 180 *L. salmonis* specimens from six locations in the North Atlantic that were collected in 2000 or 2002. We aligned our cytB (from LsSensitive and LsResistent) sequences to these 180 cytB sequences from Tjensvoll et al (2006). None of the 180 cytB sequences from historic samples had the C14065T SNP. To further validate if the C14065T SNP is unique in resistant lice we obtained samples from 9 different lice strains, including several field strains with reduced sensitivity towards deltamethrin or cis-cypermethrin.

The presented outcome of crossing resistant and sensitive lice showed that the mitochondria genome encodes the resistance. Since *L. salmonis* is ZZ (male)/Z0 (female) the maternally encoded resistance properties must be in the mtDNA. The *L. salmonis* mtDNA was characterized by Tjensvoll et al (2005) and encodes 13 proteins, two rRNAs and 22 tRNA genes.

### Genetic variation within the mtDNA

Tjensvoll et al (2006), *supra,* identified 158 haplotypes for cytb and 164 haplotypes for COI. In 19 *L. salmonis* individuals with know deltamethrin resistance only 2 haplotypes for *COI* were identified. The cytB was sequenced from 29 individuals with known deltamethrin resistance and only 1 haplotype was identified. Among 19 sensitive specimens 9 different haplotypes for cytB was present (Table 4).

**Table 4. Number of haplotypes identified for each of CytB and CoI in sensitive sea lice and resistant sea lice.**

| | **CytB** | **CoI** |
|---|---|---|
| **Tjensvoll et al** | 158 (180) | 164 (180) |
| **Sensitive lice** | 9 (19) | 11 (19) |
| **Resistant lice** | 1 (29) | 2 (19) |

### Example 3: Testing for PRLS

Based on the identification of SNPs responsible for pyrethroid resistance in *L. salmonis,* we developed several sensitive Real-Time PCR (TaqMan) 5'-nuclease assays for single nucleotide polymorphism (SNP) detection (Real-Time PCR SNP-assay) (Table 1), and successfully applied these to differentiate between pyrethroid resistant and sensitive sea lice. Fluorogenic PCR probes, chemically modified with a minor groove binding agent to increase duplex stability, were used in single and multiplex probe closed tube formats. The probes were tested in a commercially available thermocycling fluorimeter (Applied Biosystems 7500 Real-Time PCR System) at PatoGen Analyse AS laboratory in Ålesund. All assays were used qualitatively to determine the relative amount of the SNP- in individual samples, and samples from different life stages of sea lice were use. Comparison of results obtained using sea lice samples with different Real-Time PCR SNP-assays detecting different SNPs specific for pyrethroid resistant sea lice showed no discrepancies from results obtained by genome sequencing. The reported SNPs and the relevant Real-Time PCR SNP-assays are ideal for the differentiating between pyrethroid resistant and sensitive sea lice in the aquaculture industry.

### Sampling of sea lice

Sea lice samples were collected from sea lice with known sensitivity status to pyrethroids, cultivated in The Sea Lice Research Centre laboratories at the University of Bergen. The sea lice had been tested with respect to sensitivity to pyrethroids by bioassays as previously described in example 1 above. Also, the genome sequences for the relevant genes were known from previous genomic sequencing performed as described in example 1 above.

In addition, sea lice were sampled from fish farms on the west coast of Norway. Sea lice were collected using forceps, and approximately 10-50 lice per site were conserved in 70% ethanol and kept at 4°C. Samples were sent refrigerated to PatoGen by express mail carrier.

### Nucleic acid purification

In PatoGens laboratory, RNA and/or DNA were extracted from samples by methods well known to the skilled person. In short, tissue samples were transferred to Micro Collection Tubes and lysed and homogenized using QIAzol Lysis Reagent, steel beads and vigorous shaking using a TissueLyser system, followed by nucleic acid extraction using an RNAeasy kit (Qiagen) or DNAeasy kit (Qiagen), all according to the manufacturer's instructions, and by methods well known to the skilled person. Chloroform were added to the samples and shaken vigorously. After resting and centrifuging, the relevant liquid phase were collected for further extraction of either RNA or DNA by vacuum technology using a Qiagen robot system, all according to the manufacturer's instructions, and by methods well known to the skilled person. Finally, nucleic acids were eluted in 25 ml of elution buffer and used for PCR by methods well known to the skilled person.

### SNP-detection by Real-Time PCR

The primers and probes listed in table 5 are TaqMan® MGB Probe SNP Genotyping Assays using TaqMan® 5' nuclease assay chemistry for amplifying and detecting specific SNP alleles in purified genomic DNA or RNA. In this study, primers and probes SNP-14017, SNP-14065, & SNP-9035 listed in table 1 were ordered from Life Technologies Corporation. The primers and probes listed in table 1 for SNP-8605 and SNP-13957 serves as examples of assays for these SNPs, but were not included in this study. One-step amplification (45 cycles) was performed on an Applied Biosystems 7500 Real-Time PCR System performed at PatoGen Analyse AS laboratory in Ålesund, all according to the manufacturer's instructions, and by methods well known to the skilled person. All assays were tested on both RNA and DNA, and used qualitatively to determine the relative amount of the relevant SNPs in individual samples.

**Table 5: Primers and probes. Listing of primers and probes for each SNP identified. Probes are both listed with IUPAC nucleotide codes for SNPs, and with the alternative nucleotides for each position as indicated. IUPAC codes used are in accordance with Nomenclature for Incompletely Specified Bases in Nucleic Acid Sequences, of which the following are relevant here: Y = C or T, R = A or G (Nomenclature Committee of the International Union of Biochemistry (NC-IUB) (1984) http://www.chem.qmul.ac.uk/iubmb/misc/naseq.html).**

| **Assay ID** | **Position AY629857** | **Forward primer** | **Revers primer** | **Probe with IUPAC nucleotide code** | **Probe** | **Sensitive** | **Resistant** |
|---|---|---|---|---|---|---|---|
| SNP-8605 | T8605C | | | CCTCTT**Y**GAGTTTATT (SEQ ID No. 16) | | T | C |
| SNP-9035 | A9035G | | | TTCCAGGGTTTGGRTTA (SEQ ID No. 17) | | A | G |
| SNP-13957 | C13957T | | | | | C | T |
| SNP-14017 | A14017G | | | CAGCAAAAATGG**R**AA (SEQ ID No. 19 ) | | A | G |
| SNP-14065 | C14065T | | | CCCCCCYAACTTAT (SEQ ID No 20) | | C | T |

### Results

The results from Real-Time PCR-assays are interpreted by looking at the deviation in Ct-values between the probes detecting the two variants of the SNP. For the Real-Time PCR-assay towards SNP-14017 performed using RNA or DNA, sensitive lice has a deviation lower than -2, and resistant sea lice has a deviation higher than -2. For the Real-Time PCR-assays towards SNP-14065 & SNP-9035 performed using RNA or DNA, sensitive lice has a deviation lower than zero, and resistant sea lice has a deviation higher than zero. In addition, there is a quantitative aspect where the highly resistant strains have a higher deviation value than moderately resistant sea lice. Most likely, genetically resistant strains that have not been exposed to pyrethroids for some time has a lower deviation value than genetically resistant strains that been repeatedly and newly exposed to pyrethroids.

The results from the Real-Time PCR SNP-analyses using assays directed towards SNP-14065 & SNP-9035 (according to Table 5) using RNA or DNA from sea lice samples with known sensitivity to pyrethroids showed good correlation with results obtained by genome sequencing, and correlated with the known resistance status in sea lice populations (Table 6 & 7). The Real-Time PCR SNP-assay directed towards SNP-14017 (according to Table 5) gave the same results using DNA or RNA, and correlated with the results from SNP-14065 & SNP-9035 for all but one sea lice sample (Table 6).

Also, analyses performed on a higher number of sampled field strains showed good correlation with the known resistance status and as shown in Table 8. Here, analyses were performed using SNP-14065, and RNA as template.

All tested Real-Time PCR-SNP-assays show very promising results, and we believe that these SNPs can be used separately and/or in combination with other SNPs to serve as a tool in the practical differentiation between pyrethroid- sensitive and resistant sea lice.

Thus the reported SNPs and the relevant Real-Time PCR SNP-assays represent an ideal tool for differentiating between pyrethroid resistant and sensitive sea lice in the aquaculture industry. Also, the prevalence of sensitive versus resistant sea lice in a population, and the deviation value for individual lice or average deviation values for populations, can be used to predict the best possible outcome of a treatment using pyrethroids in the population. Also, the technique can become an important tool for optimizing sea lice treatments using pyrethroids, and to monitor the resistant status of populations of sea lice before treatment.

The Real-Time PCR SNP-assay directed towards SNP-14017 (according to Table 5) gave correlating results using DNA or RNA, and correlated with the results from SNP-14065 & SNP-9035 for all but one sea lice sample (sample 17).

**Table 6. Conclusions regarding resistance status showed good correlations between different assays directed towards different SNPs, and regardless of whether RNA or DNA was used as a template. The results from the Real-Time PCR SNP-analyses using assays directed towards SNP-14065 & SNP-9035 (according to Table 5) using RNA or DNA from sea lice samples with known sensitivity to pyrethroids showed full correlation.**

| **Samples from Sea lice lab at the University of Bergen** | | | **Concluding results from Real-Time PCR - SNP-analyses using RNA or DNA as template** | | | | | |
|---|---|---|---|---|---|---|---|---|
| **Kode** | **Nr** | **Lice population label** | **SNP-14017 - RNA Conclusion** | **SNP-14017 - DNA Conclusion** | **SNP-14065 - RNA Conclusion** | **SNP-14065 - DNA Conclusion** | **SNP-9035-RNA Conclusion** | **SNP-9035-DNA Conclusion** |
| AB45310871 | **1** | **A** | Resistant | Resistant | Resistant | Resistant | Resistant | Resistant |
| AB44028166 | **2** | **A** | Resistant | Resistant | Resistant | Resistant | Resistant | Resistant |
| AB38571165 | **3** | **A** | Resistant | Resistant | Resistant | Resistant | Resistant | Resistant |
| AB44589338 | **4** | **A** | Resistant | Resistant | Resistant | Resistant | Resistant | Resistant |
| AB44028181 | **5** | **A** | Resistant | Resistant | Resistant | Resistant | Resistant | Resistant |
| AB45310863 | **6** | **B** | **Sensitive** | **Sensitive** | **Sensitive** | **Sensitive** | **Sensitive** | **Sensitive** |
| AB38571150 | **7** | **B** | Resistant | Resistant | Resistant | Resistant | Resistant | Resistant |
| AB44045872 | **8** | **B** | Resistant | Resistant | Resistant | Resistant | Resistant | Resistant |
| AB44028185 | **9** | **B** | Resistant | Resistant | Resistant | Resistant | Resistant | Resistant |
| AB45310870 | **10** | **B** | **Sensitive** | **Sensitive** | **Sensitive** | **Sensitive** | **Sensitive** | **Sensitive** |
| AB45310855 | **11** | **B** | Resistant | Resistant | Resistant | Resistant | Resistant | Resistant |
| AB45313574 | **12** | **C** | **Sensitive** | **Sensitive** | **Sensitive** | **Sensitive** | **Sensitive** | **Sensitive** |
| AB38571164 | **13** | **C** | Resistant | Resistant | Resistant | Resistant | Resistant | Resistant |
| AB45313582 | **14** | **C** | Resistant | Resistant | Resistant | Resistant | Resistant | Resistant |
| AB45310865 | **15** | **C** | Resistant | Resistant | Resistant | Resistant | Resistant | Resistant |
| AB45428417 | **16** | **C** | **Sensitive** | **Sensitive** | **Sensitive** | **Sensitive** | **Sensitive** | **Sensitive** |
| AB45310852 | **17** | **C** | Resistant | Resistant | **Sensitive** | **Sensitive** | **Sensitive** | **Sensitive** |
| AB45310038 | **18** | **C** | **Sensitive** | **Sensitive** | **Sensitive** | **Sensitive** | **Sensitive** | **Sensitive** |
| AB45666543 | **19** | **D** | Resistant | Resistant | Resistant | Resistant | Resistant | Resistant |
| AB44045871 | **20** | **D** | **Sensitive** | **Sensitive** | **Sensitive** | **Sensitive** | **Sensitive** | **Sensitive** |
| AB44589289 | **21** | **D** | **Sensitive** | **Sensitive** | **Sensitive** | **Sensitive** | **Sensitive** | **Sensitive** |
| AB44506537 | **22** | **D** | **Sensitive** | **Sensitive** | **Sensitive** | **Sensitive** | **Sensitive** | **Sensitive** |
| AB45108831 | **23** | **D** | **Sensitive** | **Sensitive** | **Sensitive** | **Sensitive** | **Sensitive** | **Sensitive** |
| AB44028226 | **24** | **D** | **Sensitive** | **Sensitive** | **Sensitive** | **Sensitive** | **Sensitive** | **Sensitive** |
| AB45312384 | **25** | **E** | **Sensitive** | **Sensitive** | **Sensitive** | **Sensitive** | **Sensitive** | **Sensitive** |
| AB44001158 | **26** | **E** | **Sensitive** | **Sensitive** | **Sensitive** | **Sensitive** | **Sensitive** | **Sensitive** |
| AB44035432 | **27** | **E** | **Sensitive** | **Sensitive** | **Sensitive** | **Sensitive** | **Sensitive** | **Sensitive** |
| AB45312386 | **28** | **E** | **Sensitive** | **Sensitive** | **Sensitive** | **Sensitive** | **Sensitive** | **Sensitive** |
| AB44506534 | **29** | **E** | **Sensitive** | **Sensitive** | **Sensitive** | **Sensitive** | **Sensitive** | **Sensitive** |
| AB44589313 | **30** | **E** | **Sensitive** | **Sensitive** | **Sensitive** | **Sensitive** | **Sensitive** | **Sensitive** |
| AB45313587 | **31** | **E** | **Sensitive** | **Sensitive** | **Sensitive** | **Sensitive** | **Sensitive** | **Sensitive** |
| AB45428414 | 32 | F | Resistant | Resistant | Resistant | Resistant | Resistant | Resistant |
| AB41018802 | **33** | **F** | Resistant | Resistant | Resistant | Resistant | Resistant | Resistant |
| AB44589322 | **34** | **F** | Resistant | Resistant | Resistant | Resistant | Resistant | Resistant |
| AB44035431 | **35** | **F** | Resistant | Resistant | Resistant | Resistant | Resistant | Resistant |
| AB45310868 | **36** | **F** | Resistant | Resistant | Resistant | Resistant | Resistant | Resistant |
| AB45309850 | **37** | **G** | Resistant | Resistant | Resistant | Resistant | Resistant | Resistant |
| AB41018811 | **38** | **G** | Resistant | Resistant | Resistant | Resistant | Resistant | Resistant |
| AB44594130 | **39** | **G** | Resistant | Resistant | Resistant | Resistant | Resistant | Resistant |
| AB45310851 | **40** | **G** | Resistant | Resistant | Resistant | Resistant | Resistant | Resistant |
| AB44506582 | **41** | **G** | Resistant | Resistant | Resistant | Resistant | Resistant | Resistant |
| AB45443862 | **42** | **G** | Resistant | Resistant | Resistant | Resistant | Resistant | Resistant |
| AB41018799 | **43** | **G** | Resistant | Resistant | Resistant | Resistant | Resistant | Resistant |

**Table 7. SNP-analyses correlate well with known resistance status. SNP-analyses using SNP14065 correlates with known resistance status in sea lice (L. salmonis) from the sea lice lab at the University of Bergen (based on bioassays and field observations), and with genotyping performed by the University of Bergen. Bioassays, genotyping and SNP-analyses are all based on different lice from the same populations, and thus the results cannot be correlated on individual sea lice level. Also, since the number of samples is very low, the slight differences with respect to prevalence of resistant genotype observed between sequencing and Real-Time PCR SNP-14065-analysis must be attributed to the low number of samples tested, and this is especially the case for lice population D. Still, the conclusions with respect to population resistance level correlates well. Also, populations A, B and C represent surviving sea lice from the same original population, but A, B and C are surviving lice that has been exposed to different concentrations of pyrethroid in a bioassay. The results show that pyrethroid treatment selects for more resistant sea lice, and this is reflected in both the prevalence of genetically resistant lice, and in the total average deviation in Ct-values between the probes detecting the two variants of the SNP.**

| **Results from genotyping CytB - 14065** | | | **Results from genotyping CytB - 14065** | | | | | |
|---|---|---|---|---|---|---|---|---|
| **Lice population label** | **Isolate description/ bio-assay conclusions** | | **# tested** | | **# Resistant** | | **# Sensitive** | **Prevalence of resistant** |
| A | Surviving lice from bioassay | Highest dose | 5 | | 5 | | 0 | 100 |
| B | Surviving lice from bioassay | Second highest dose | 2 | | 1 | | 1 | 50 |
| C | Surviving lice from bioassay | Control | 6 | | 2 | | 4 | 33 |
| D | HF | Slightly reduced sensitivity | 3 | | 2 | | 1 | 67 |
| E | LG | Sensitive | 3 | | 0 | | 3 | 0 |
| F | LF | Resistant | 13 | | 12 | | 1 | 92 |
| G | LB | Multi-resistant | 10 | | 10 | | 0 | 100 |

| **Samples from Sea lice lab at the University of Bergen** | | | **Results Real-Time PCR - SNP-14065** | | | | | |
|---|---|---|---|---|---|---|---|---|
| **Lice population label** | **Isolate description/ bio-assay conclusions** | | **# tested** | **# Resistant** | | **# Sensitive** | **% prevalence sensitive** | **% prevalence resistant** |
| A | Surviving lice from | Highest dose | 5 | 5 | | 0 | 0 | 100 |
| | bioassay | | | | | | | |
| B | Surviving lice from bioassay | Second highest dose | 6 | 4 | | 2 | 33 | 67 |
| C | Surviving lice from bioassay | Control | 7 | 3 | | 4 | 57 | 43 |
| D | HF | Slightly reduced sensitivity | 6 | 1 | | 5 | 83 | 17 |
| E | LG | Sensitive | 7 | 0 | | 7 | 100 | 0 |
| F | LF | Resistant | 5 | 5 | | 0 | 0 | 100 |
| G | LB | Multi-resistant | 7 | 7 | | 0 | 0 | 100 |

| **Samples from Sea lice lab at the University of Bergen** | | | **Results Real-Time PCR - SNP-14065** | | | | | |
|---|---|---|---|---|---|---|---|---|
| **Lice population label** | **Isolate description/ bio-assay conclusions** | | **Average deviation sensitive** | | **Average deviation resistant** | | **Total average deviation** | **Conclusion resistance status** |
| A | Surviving lice from bioassay | Highest dose | - | | 12,9 | | 12,9 | Resistant |
| B | Surviving lice from bioassay | Second highest dose | -1,5 | | 13,7 | | 8,63 | Resistant |
| C | Surviving lice from bioassay | Control | -1,6 | | 12,6 | | 4,53 | Reduced sensitivity |
| D | HF | Slightly reduced sensitivity | -1,3 | | 12,9 | | 1,03 | Slightly reduced sensitivity |
| E | LG | Sensitive | -1,4 | | | | -1,36 | Sensitive |
| F | LF | Resistant | - | | 11,8 | | 11,84 | Resistant |
| G | LB | Multi-resistant | - | | 10,3 | | 10,34 | Resistant |

**Table 8. SNP-analyses correlate well with known resistance status in field strains. Field strains of sea lice (L. salmonis) were characterized with respect to sensitivity to pyrethroids at the University of Bergen sea lice lab, and with the exception of strain P, all strains have been cultivated for one or more generations in the lab (up to 34 generations) before sampling. The conclusions from Real-Time PCR analyses towards SNP-14065 correlates very well with the known resistance characteristics from bioassays and field observations.**

| **Samples from Sea lice lab at the University of Bergen** | | **Results Real-Time PCR - SNP-14065** | | | | | | | | |
|---|---|---|---|---|---|---|---|---|---|---|
| **Lice population label/ bio-assay conclusions** | | **n** | **# Resistant** | **# Sensitive** | **% prevalence sensitive** | **% prevalence resistant** | **Average deviation sensitive** | **Average deviation resistant** | **Total average deviation** | **Conclusion resistance status** |
| H | Sensitive | 30 | 0 | 30 | 100 | 0 | -1,7 | - | -1,7 | Sensitive |
| I | Resistant | 30 | 30 | 0 | 0 | 100 | - | 5,5 | 5,5 | Reduced sensitivity |
| J | Resistant | 30 | 30 | 0 | 0 | 100 | - | 5,1 | 5,1 | Reduced sensitivity |
| K | Sensitive | 30 | 0 | 30 | 100 | 0 | -1,3 | - | -1,3 | Sensitive |
| L | Sensitive | 30 | 0 | 30 | 100 | 0 | -1,7 | - | -1,7 | Sensitive |
| M | Sensitive | 30 | 0 | 30 | 100 | 0 | -1,4 | - | -1,4 | Sensitive |
| N | Sensitive | 7 | 0 | 7 | 100 | 0 | -1,4 | - | -1,4 | Sensitive |
| O | Sensitive | 28 | 0 | 28 | 100 | 0 | -1,3 | - | -1,3 | Sensitive |
| P | Slightly reduced sensitivity | 30 | 1 | 29 | 97 | 3 | -0,7 | 10,7 | -0,3 | Sensitive (Slightly reduced sensitivity) |

## Claims

1. An in vitro method for detection of pyrethroid resistance in one or more crustaceans, comprising the steps of
a. analyzing the mitochondrial genomic material of the crustacean,
b. detecting one or more single nucleotide polymorphisms (SNP) associated with pyrethroid resistance in the mitochondrial DNA of the crustacean to be analyzed.

2. A method according to claim 1, wherein the crustaceans is one or more copepods, preferably a copepod belonging to the family Caligidae, more preferably a copepod selected from the group consisting of *Lepeophteirus salmonis, Caligus elongatus,* and *Caligus rogercresseyi.*

3. A method according to claim 2, comprising the steps of detecting single nucleotide polymorphism (SNP) associated with pyrethroid resistance in the mitochondrial genome of a sea lice to be analyzed, wherein said sea lice is resistant to pyrethroids if at least one of the following nucleotides:
i. C in position 8605;
ii. G in position 9035;
iii. T in position 13957;
iv. G in position 14017;
v. T in position 14065;
or the complementary oligonucleotide thereof,
is present in the mitochondrial genome sequence of the one or more sea lice to be analyzed, and wherein the numbering of said positions is in accordance with the sequence depicted in SEQ ID No. 1.

4. A method according to any of the claims 1-3, wherein the crustacean is a sea lice, and further comprising the steps of:
a) collecting sea lice from infested fish or water samples;
b) isolating mitochondrial genomic material from any life stage of collected sea lice
c) determining the nucleotide polymorphic site at the positions 8605, 9035 and 14065 of the isolated mitochondrial DNA compared with of the mitochondrial nucleic acid sequence SEQ ID No. 1, wherein said sea lice is resistant to pyrethroids if at least one of the nucleotide C, G, T, G and T (U), or a complementary oligonucleotide thereof, is present in position 8605, 9035, 13957, 14017, and 14065, respectively.

5. A method according to claim 4, wherein step c) is performed using a primer selected from the group consisting of SEQ ID No. 6-15 or wherein step c) is performed using at least one probe selected from the group consisting of SEQ ID No. 16-20.

6. A method according to any of claims 4-5, wherein step c) is performed by contacting mitochondrial DNA sequence of the sea lice to be analyzed with a detection reagent, and determining which nucleotide is present in position 8605, 9035, 13957, 14017 and 14065.

7. An isolated oligonucleotide sequence comprising at least one single-nucleotide polymorphism (SNP) associated with pyrethroid resistance in crustaceans, wherein said isolated oligonucleotide sequence comprises nucleotides that distinguishes crustaceans which are resistant to pyrethroids from non-resistance to pyrethroids, and wherein the SNPs are present in the mitochondrial DNA of the crustaceans, and wherein said isolated oligonucleotide sequence comprises at least 8 contiguous nucleotides of the sequence SEQ ID No. 5, or complementary sequences of SEQ ID No. 5, provided that at least one SNP selected from the group of SNPs consisting of T8605C (U8605C in case of RNA), C13957T (U13957T in case of RNA), and C14065T (C14065U in case of RNA) is present in said isolated oligonucleotide sequence, and wherein the numbering of said positions is in accordance with the sequence depicted in SEQ ID No. 1.

8. An isolated oligonucleotide sequence according to claim 7, wherein said isolated oligonucleotide sequence comprises at least 20 contiguous nucleotides.

9. An isolated oligonucleotide sequence complementary to the oligonucleotide sequence according to claim 7, wherein said at least one SNP selected from the group of SNPs consisting of T8605C (U8605C in case of RNA), C13957T (U13957T in case of RNA), and C14065T (C14065U in case of RNA) is present, or a complementary oligonucleotide thereof and wherein the numbering of said positions is in accordance with the sequence depicted in SEQ ID No. 1.

10. An isolated oligonucleotide sequence according to any of claims 7-9, wherein the crustacean is a copepod, preferably a copepod belonging to the family Caligidae, more preferably a copepod selected from the group consisting of *Lepeophteirus salmonis, Caligus elongatus,* and *Caligus rogercresseyi.*

11. An isolated oligonucleotide sequence comprising at least one single-nucleotide polymorphism (SNP) associated with pyrethroid resistance in crustaceans, wherein said isolated oligonucleotide sequence comprises nucleotides that distinguishes crustaceans which are resistant to pyrethroids from non-resistance to pyrethroids, and wherein the SNPs are present in the mitochondrial DNA of the crustaceans, and wherein said oligonucleotide sequence comprises 12 to 25 consecutive nucleotides of SEQ ID No. 4, or complementary sequences of SEQ ID No. 4, provided that at least one SNP selected from the group of SNPs consisting of T8605C (U8605C in case of RNA), A9035G, C13957T (U13957T in case of RNA), and C14065T (C14065U in case of RNA) is present in said isolated oligonucleotide sequence, and wherein the numbering of said positions is in accordance with the sequence depicted in SEQ ID No. 1.

12. A oligonucleotide probe for detection of pyrethroid resistance in crustaceans, preferably copepods, more preferably in sea lice (Lepeophtheirus salmonis), wherein the sequence of said probe is selected from the group consisting of SEQ ID No. 16, SEQ ID No. 17, SEQ ID No. 18, and SEQ ID No. 20, and sequences having over their entire length at least 80 % sequence identity therewith, or a complementary sequence thereof.

13. A oligonucleotide primer for detection of pyrethroid resistance in crustaceans, preferably copepods, more preferably in sea lice (Lepeophtheirus salmonis), wherein the sequence of said primer is selected from the group consisting of SEQ ID No. 6, SEQ ID No. 7, SEQ ID No. 8, SEQ ID No. 9, SEQ ID No. 10, SEQ ID No. 11, and SEQ ID No. 14, and SEQ ID No. 15, and sequences having over their entire length at least 80 % sequence identity therewith, or a complementary sequence thereof.

14. A kit for detection of pyrethroid resistance in crustaceans, preferably copepods, more preferably in sea lice (*Lepeoptheirus salmonis*), which comprises at least one oligonucleotide sequence or probe or primer according to any of the claims 7-13.

15. An in vitro use of an isolated oligonucleotide sequence comprising at least 8 contiguous nucleotides of the sequence selected from the group consisting of SEQ ID No. 4 or SEQ ID No. 5, wherein said sequence comprises at least one of the nucleotide C, G, T, G and T (U), or a complementary oligonucleotide thereof, is present in position 8605, 9035, 13957, 14017, and 14065, respectively, and wherein the numbering of said positions is in accordance with the sequence depicted in SEQ ID No. 1, and wherein the sequences are used for detection of pyrethroid resistance in crustaceans.

## Patentansprüche

1. In-vitro-Verfahren zur Detektion einer Pyrethroidresistenz in einem oder mehreren Krebstieren, umfassend die folgenden Schritte:
a) Analysieren des mitochondrialen genomischen Materials des Krebstiers,
b) Detektieren von einem oder mehreren mit einer Pyrethroidresistenz assoziierten Einzelnukleotid-Polymorphismen (SNP) in der zu analysierenden mitochondrialen DNA des Krebstiers.

2. Verfahren nach Anspruch 1, wobei es sich bei den Krebstieren um einen oder mehrere Ruderfußkrebse handelt, vorzugsweise einen Ruderfußkrebs, der zur Familie Caligidae gehört, stärker bevorzugt einen Ruderfußkrebs, der aus der Gruppe ausgewählt ist, die aus *Lepeophteirus salmonis, Caligus elongatus* und *Caligus rogercresseyi* besteht.

3. Verfahren nach Anspruch 2, umfassend die Schritte des Detektierens eines mit einer Pyrethroidresistenz assoziierten Einzelnukleotid-Polymorphismus (SNP) in dem zu analysierenden mitochondrialen Genom einer Fischlaus, wobei die Fischlaus resistent gegenüber Pyrethroiden ist, wenn wenigstens eines der folgenden Nukleotide:
i. C an Position 8605;
ii. G an Position 9035;
iii. T an Position 13957;
iv. G an Position 14017;
v. T an Position 14065;
oder das komplementäre Oligonukleotid davon,
in der zu analysierenden mitochondrialen Genomsequenz der einen oder mehreren Fischläuse vorhanden ist, und wobei die Nummerierung der Positionen in Einklang mit der in SEQ ID Nr. 1 abgebildeten Sequenz steht.

4. Verfahren nach einem der Ansprüche 1 bis 3, wobei das Krebstier eine Fischlaus ist, und ferner umfassend die folgenden Schritte:
a) Sammeln von Fischläusen von befallenen Fischen oder Wasserproben;
b) Isolieren von mitochondrialem genomischem Material aus jedem Lebensstadium der gesammelten Fischläuse;
c) Bestimmen der Nukleotidpolymorphismus-Stelle an den Positionen 8605, 9035 und 14065 der isolierten mitochondrialen DNA im Vergleich zur mitochondrialen Nukleinsäuresequenz SEQ ID Nr. 1, wobei die Fischlaus resistent gegenüber Pyrethroiden ist, wenn wenigstens eines der Nukleotide C, G, T, G und T (U) oder ein komplementäres Oligonukleotid davon an Position 8605, 9035, 13957, 14017 bzw. 14065 vorhanden ist.

5. Verfahren nach Anspruch 4, wobei der Schritt c) unter Verwendung eines Primers durchgeführt wird, der aus der Gruppe bestehend aus SEQ ID Nr. 6 bis 15 ausgewählt wird, oder wobei der Schritt c) unter Verwendung von wenigstens einer Sonde durchgeführt, wird die aus der Gruppe bestehend aus SEQ ID Nr. 16 bis 20 ausgewählt wird.

6. Verfahren nach einem der Ansprüche 4 bis 5, wobei der Schritt c) durchgeführt wird, indem eine zu analysierende mitochondriale DNA-Sequenz der Fischlaus mit einem Detektionsreagens in Kontakt gebracht wird und bestimmt wird, welches Nukleotid an der Position 8605, 9035, 13957, 14017 und 14065 vorhanden ist.

7. Isolierte Oligonukleotidsequenz, umfassend wenigstens einen mit Pyrethroidresistenz in Krebstieren assoziierten Einzelnukleotid-Polymorphismus (SNP), wobei die isolierte Oligonukleotidsequenz Nukleotide umfasst, die Krebstiere, die resistent gegenüber Pyrethroiden sind, von einer Nichtresistenz gegenüber Pyrethroiden unterscheiden, und wobei die SNPs in der mitochondrialen DNA der Krebstiere vorhanden sind, und wobei die isolierte Oligonukleotidsequenz wenigstens 8 aneinandergrenzende Nukleotide der Sequenz SEQ ID Nr. 5 oder komplementäre Sequenzen von SEQ ID Nr. 5 umfasst, mit der Maßgabe, dass wenigstens ein SNP, ausgewählt aus der Gruppe von SNPs, die aus T8605C (U8605C im Fall von RNA), C13957T (U13957T im Fall von RNA) und C14065T (C14065U im Fall von RNA) besteht, in der isolierten Oligonukleotidsequenz vorhanden ist, und wobei die Nummerierung der Positionen in Einklang mit der in SEQ ID Nr. 1 abgebildeten Sequenz steht.

8. Isolierte Oligonukleotidsequenz nach Anspruch 7, wobei die isolierte Oligonukleotidsequenz wenigstens 20 aneinandergrenzende Nukleotide umfasst.

9. Isolierte Oligonukleotidsequenz, die komplementär zu der Oligonukleotidsequenz nach Anspruch 7 ist, wobei der mindestens eine SNP ausgewählt aus der Gruppe von SNPs, die aus T8605C (U8605C im Fall von RNA), C13957T (U13957T im Fall von RNA) und C14065T (C14065U im Fall von RNA) besteht, in der isolierten Oligonukleotidsequenz vorhanden ist, oder ein komplementäres Oligonukleotid davon umfasst, und wobei die Nummerierung der Positionen in Einklang mit der in SEQ ID Nr. 1 abgebildeten Sequenz steht.

10. Isolierte Oligonukleotidsequenz nach einem der Ansprüche 7 bis 9, wobei das Krebstier ein Ruderfußkrebs ist, vorzugsweise ein Ruderfußkrebs, der zur Familie Caligidae gehört, stärker bevorzugt ein Ruderfußkrebs, der aus der Gruppe ausgewählt ist, die aus *Lepeophteirus salmonis, Caligus elongatus* und *Caligus rogercresseyi* besteht.

11. Isolierte Oligonukleotidsequenz, umfassend wenigstens einen mit einer Pyrethroidresistenz assoziierten Einzelnukleotid-Polymorphismus (SNP) in Krebstieren, wobei die isolierte Oligonukleotidsequenz Nukleotide umfasst, die Krebstiere die resistent gegenüber Pyrethroiden sind, von einer Nichtresistenz gegenüber Pyrethroiden unterscheiden, und wobei die SNPs in der mitochondrialen DNA der Krebstiere vorhanden sind, und wobei die Oligonukleotidsequenz 12 bis 25 aneinandergrenzende Nukleotide von SEQ ID Nr. 4 oder komplementär Sequenzen von SEQ ID Nr. 4 umfasst, mit der Maßgabe, dass wenigstens ein SNP, ausgewählt aus der Gruppe von SNPs, die aus T8605C (U8605C im Fall von RNA), A9035G, C13957T (U13957T im Fall von RNA) und C14065T (C14065U im Fall von RNA) besteht, in der isolierten Oligonukleotidsequenz vorhanden ist, und wobei die Nummerierung der Positionen in Einklang mit der in SEQ ID Nr. 1 abgebildeten Sequenz steht.

12. Oligonukleotid-Sonde zur Detektion einer Pyrethroidresistenz in Krebstieren, vorzugsweise in Ruderfußkrebsen, stärker bevorzugt in Fischläusen (*Lepeoptheirus salmonis),* wobei die Sequenz der Sonde aus der Gruppe ausgewählt ist, die aus Folgendem besteht: SEQ ID Nr. 16, SEQ ID Nr. 17, SEQ ID Nr. 18 und SEQ ID Nr. 20 sowie Sequenzen, die über ihre gesamte Länge wenigstens 80 % Sequenzidentität damit aufweisen, oder einer komplementären Sequenz davon.

13. Oligonukleotid-Primer zur Detektion einer Pyrethroidresistenz in Krebstieren, vorzugsweise in Ruderfußkrebsen, stärker bevorzugt in Fischläusen *(Lepeoptheirus salmonis),* wobei die Sequenz des Primers aus der Gruppe ausgewählt ist, die aus Folgendem besteht SEQ ID Nr. 6, SEQ ID Nr. 7, SEQ ID Nr. 8, SEQ ID Nr. 9, SEQ ID Nr. 10, SEQ ID Nr. 11 und SEQ ID Nr. 14 und SEQ ID Nr. 15 sowie Sequenzen, die über ihre gesamte Länge wenigstens 80 % Sequenzidentität damit aufweisen, oder einer komplementären Sequenz davon.

14. Kit zur Detektion einer Pyrethroidresistenz in Krebstieren, vorzugsweise in Ruderfußkrebsen, stärker bevorzugt in Fischläusen (*Lepeoptheirus salmonis*), das wenigstens eine Oligonukleotidsequenz oder eine Sonde oder einen Primer nach einem der Ansprüche 7 bis 13 umfasst.

15. In-Vitro-Verwendung einer isolierten Oligonukleotidsequenz, umfassend wenigstens 8 aneinandergrenzende Nukleotide der Sequenz, ausgewählt aus der Gruppe bestehend aus SEQ ID Nr. 4 oder SEQ ID Nr. 5, wobei die Sequenz wenigstens eines der Nukleotide C, G, T, G und T (U) oder ein komplementäres Oligonukleotid davon umfasst, das an der Position 8605, 9035, 13957, 14017 bzw. 14065 vorhanden ist, und wobei die Nummerierung der Positionen in Einklang mit der in SEQ ID Nr. 1 abgebildeten Sequenz steht und wobei die Sequenzen zur Detektion einer Pyrethroidresistenz in Krebstieren verwendet werden.

## Revendications

1. Procédé *in vitro* pour la détection d'une résistance aux pyréthroïdes chez un ou plusieurs crustacés, comprenant les étapes consistant à
a. analyser le matériel génomique mitochondrial du crustacé,
b. détecter un ou plusieurs polymorphismes nucléotidiques uniques (SNP) associés à une résistance aux pyréthroïdes dans l'ADN mitochondrial du crustacé devant être analysé.

2. Procédé selon la revendication 1, dans lequel le crustacé est un ou plusieurs copépodes, de préférence un copépode appartenant à la famille des Caligidae, de préférence encore un copépode choisi dans le groupe constitué par *Lepeophteirus salmonis, Caligus elongatus* et *Caligus rogercresseyi.*

3. Procédé selon la revendication 2, comprenant les étapes consistant à détecter un polymorphisme nucléotidique unique (SNP) associé à une résistance aux pyréthroïdes dans le génome mitochondrial d'un pou de poisson devant être analysé, dans lequel ledit pou de poisson est résistant aux pyréthroïdes si au moins l'un des nucléotides suivants :
i. C en position 8605 ;
ii. G en position 9035 ;
iii. T en position 13957 ;
iv. G en position 14017 ;
v. T en position 14065 ;
ou l'oligonucléotide complémentaire de ceux-ci,
est présent dans la séquence génomique mitochondriale desdits un ou plusieurs poux de poisson devant être analysés, et dans lequel la numérotation desdites positions est conforme à la séquence décrite dans SEQ ID NO: 1.

4. Procédé selon l'une quelconque des revendications 1 à 3, dans lequel le crustacé est un pou de poisson, et comprenant en outre les étapes consistant à :
a) recueillir des poux de poisson à partir de poissons ou d'échantillons d'eau infestés ;
b) isoler le matériel génomique mitochondrial à partir de tout stade de vie des poux de poisson récoltés
c) déterminer le site nucléotidique polymorphique aux positions 8605, 9035 et 14065 de l'ADN mitochondrial isolé en comparant avec la séquence d'acide nucléique mitochondrial SEQ ID NO: 1, dans lequel ledit pou de poisson est résistant aux pyréthroïdes si au moins l'un des nucléotides C, G, T, G et T (U), ou un oligonucléotide complémentaire de ceux-ci, est présent en position 8605, 9035, 13957, 14017 et 14065, respectivement.

5. Procédé selon la revendication 4, dans lequel l'étape c) est mise en œuvre en utilisant une amorce choisie dans le groupe constitué par SEQ ID NO: 6 à 15, ou dans lequel l'étape c) est mise en œuvre en utilisant au moins une sonde choisie dans le groupe constitué par SEQ ID NO: 16 à 20.

6. Procédé selon l'une quelconque des revendications 4-5, dans lequel l'étape c) est mise en œuvre en mettant en contact une séquence d'ADN mitochondrial du pou de poisson devant être analysé avec un réactif de détection, et en déterminant quel nucléotide est présent en position 8605, 9035, 13957, 14017 et 14065.

7. Séquence oligonucléotidique isolée comprenant au moins un polymorphisme nucléotidique unique (SNP) associé à une résistance aux pyréthroïdes *chez des crustacés,* laquelle séquence oligonucléotidique isolée comprend des nucléotides qui distinguent les crustacés qui sont résistants aux pyréthroïdes d'une non-résistance aux pyréthroïdes, et dans laquelle les SNP sont présents dans l'ADN mitochondrial des crustacés, et laquelle séquence oligonucléotidique isolée comprend au moins 8 nucléotides contigus de la séquence SEQ ID NO: 5, ou des séquences complémentaires de SEQ ID NO: 5, dans lequel ledit au moins un SNP choisi dans le groupe des SNP consistant en T8605C (U8605C dans le cas d'un ARN), C13957T (U13957T dans le cas d'un ARN) et C14065T (C14065U dans le cas d'un ARN) est présent dans ladite séquence oligonucléotidique isolée, et dans laquelle la numérotation desdites positions est conforme à la séquence décrite dans SEQ ID NO: 1.

8. Séquence oligonucléotidique isolée selon la revendication 7, laquelle séquence oligonucléotidique isolée comprend au moins 20 nucléotides contigus.

9. Séquence oligonucléotidique isolée complémentaire de la séquence oligonucléotide selon la revendication 7, étant entendu qu'au moins un SNP choisi dans le groupe des SNP consistant en T8605C (U8605C dans le cas d'un ARN), C13957T (U13957T dans le cas d'un ARN) et C14065T (C14065U dans le cas d'un ARN) est présent dans ladite séquence oligonucléotidique isolée, ou un oligonucléotide complémentaire de celle-ci, et dans laquelle la numérotation desdites positions est conforme à la séquence décrite dans SEQ ID NO: 1.

10. Séquence oligonucléotidique isolée selon l'une quelconque des revendications 7 à 9, dans laquelle le crustacé est un copépode, de préférence un copépode appartenant à la famille des Caligidae, de préférence encore un copépode choisi dans le groupe constitué par *Lepeophteirus salmonis, Caligus elongatus* et *Caligus rogercresseyi.*

11. Séquence oligonucléotidique isolée comprenant au moins un polymorphisme nucléotidique unique (SNP) associé à une résistance aux pyréthroïdes *chez des crustacés,* laquelle séquence oligonucléotidique isolée comprend des nucléotides qui distinguent les crustacés qui sont résistants aux pyréthroïdes d'une non-résistance aux pyréthroïdes, et dans laquelle les SNP sont présents dans l'ADN mitochondrial des crustacés, et laquelle séquence oligonucléotidique isolée comprend 12 à 25 nucléotides contigus de la séquence SEQ ID NO: 4, ou des séquences complémentaires de SEQ ID NO: 4, étant entendu qu'au moins un SNP choisi dans le groupe des SNP consistant en T8605C (U8605C dans le cas d'un ARN), A9035G, C13957T (U13957T dans le cas d'un ARN) et C14065T (C14065U dans le cas d'un ARN) est présent dans ladite séquence oligonucléotidique isolée, et dans laquelle la numérotation desdites positions est conforme à la séquence décrite dans SEQ ID NO: 1.

12. Sonde oligonucléotidique pour la détection d'une résistance aux pyréthroïdes chez des crustacés, de préférence les copépodes, de préférence encore chez des poux de poisson (*Lepeophteirus salmonis*), dans laquelle la séquence de ladite sonde est choisie dans le groupe constitué par SEQ ID NO: 16, SEQ ID NO: 17, SEQ ID NO: 18 et SEQ ID NO: 20, et les séquences ayant sur la totalité de leur longueur au moins 80 % d'identité de séquences avec celles-ci, ou une séquence complémentaire de celles-ci.

13. Amorce oligonucléotidique pour la détection d'une résistance aux pyréthroïdes chez des crustacés, de préférence les copépodes, de préférence encore chez des poux de poisson (*Lepeophteirus salmonis*), dans laquelle la séquence de ladite amorce est choisie dans le groupe constitué par SEQ ID NO: 6, SEQ ID NO: 7, SEQ ID NO: 8, SEQ ID NO: 9, SEQ ID NO: 10, SEQ ID NO: 11 et SEQ ID NO: 14 et SEQ ID NO: 15, et les séquences ayant sur la totalité de leur longueur au moins 80 % d'identité de séquences avec celles-ci, ou une séquence complémentaire de celles-ci.

14. Kit pour la détection d'une résistance aux pyréthroïdes chez des crustacés, de préférence les copépodes, de préférence encore chez les poux de poisson (*Lepeophteirus salmonis*), qui comprend au moins une séquence oligonucléotidique ou sonde ou amorce selon l'une quelconque des revendications 7 à 13.

15. Utilisation *in vitro* d'une séquence oligonucléotidique isolée comprenant au moins 8 nucléotides contigus de la séquence choisie dans le groupe constitué par SEQ ID NO: 4 ou SEQ ID NO: 5, dans laquelle ladite séquence comprend au moins l'un des nucléotides C, G, T, G et T (U), ou un oligonucléotide complémentaire de ceux-ci, présent en position 8605, 9035, 13957, 14017 et 14065, respectivement, et dans laquelle la numérotation desdites positions est conforme à la séquence décrite dans SEQ ID NO: 1, et dans laquelle les séquences sont utilisées pour la détection d'une résistance aux pyréthroïdes chez des crustacés.


### SEQUENCE LISTING

<110> PatoGen Analyse AS
<120> method for detection of pyrethroid resistance in crustaceans and
   oligonucleotide sequences useful in detection of pyrethroid resistance
<130> P23343PC00
<160> 20
<170> PatentIn version 3.5
<210> 1
   <211> 15445
   <212> DNA
   <213> Lepeophtheirus salmonis
<400> 1
<210> 2
   <211> 1056
   <212> DNA
   <213> Lepeophtheirus salmonis
<400> 2
   ctgtctcagc cgggggcata tttaggggat tcccaggttt ataatgttat tgtaactgct        60
   catgccttca ttataatctt tttcatagta atacctgtat taattggggg gtttggaaat       120
   tgattagttc ctttaatact gggggcacct gacatagctt tccctcgctt aaacaatata       180
   agattttggt ttttaatacc ctctttgagt ttattggtta taagggcatt agtagaaagt       240
   ggtgcaggaa ctgggtgaac agtatacccc cccctgtctt cgggagtttt tcactccggg       300
   gcttcagtag attttgcgat tttttccctc cacttagctg gagtgtcttc tttattaggg       360
   gctgtaaact ttattagaac tattacaaat ttacggtgct tgggactttt agtggggcaa       420
   atgccaatat tcccctgatc cgttttaatc actgctgtgc ttttactctt gtctttacca       480
   gtgttagcgg gagcaattac tatacttctt actgatcgaa atttaaatac aacttttttt       540
   gaccctagag gtggagggga tcccatttta taccagcatt tattttgatt tttcgggcac        600
   cctgaagttt atattctaat tcttccaggg tttggattaa tctctcagat tattacccaa       660
   gaaacttgta aagatgaggc ttttggttcg cttggtataa tttatgcaat agtagcaatt       720
   ggagtattgg gatttattgt ttgagctcat cacatgttta cagttgggtt agactcagac       780
   actcgagcgt attttacggc agccactata gtaattgcta tccctacagg gattaaagta       840
   tttagttgat tagggccttt atatggaagt aagattattt ataccccctc aatttattgg       900
   gtggtagggt ttctgtttct ttttacagtg gggggattaa caggtattgt attagctaac       960
   tctgccttgg atatgacttt gcatgacact tactatgtag ttgcccactt tcactatgtt      1020
   cttagaatag gagcagtttt tcgacctagg cttagg                               1056
<210> 3
   <211> 996
   <212> DNA
   <213> Lepeophtheirus salmonis
<400> 3
   ttttaaccag caaaataatc taattattta tgtttgtgta atgcgggcag ttattacgcc      60
   tgtcaaatga cttcaaacta ataaaagttt agctcacaat ccgtctgagt aaggtaagga     120
   cagccctact ctgaagtata agacagtaaa aacttggccc aggaaaatat agtttccctc     180
   tacaggtttt gctccaatcc aggttaaaag aaaaaaaatc ccgataaaag ctcaaaacat     240
   aagcattttc gaggggttaa atgtagcagg tacggcggtt cttttatccc ccagcaaaaa     300
   tggaaaaaaa aacaaaattc ttacagacaa agctaaagcc actacccccc ccaacttatt     360
   tgggatagac cgcaaaatag cataagcaaa caaaaaatac cattcaggtt gaatgtgagc     420
   aggagttact aaaggattag ctggaataaa gttttcaggg tctgctaata atcagggaga     480
   gtaagaaact aaagctccca tgagacaaaa aataagaccg aagccaaagg cgtctttaaa     540
   ggtatagtaa gggtcaaaag gaatcttata atagtttcct ggaaccccca atggatttga     600
   ggaccctgtt gaatgaagaa aaattgtatg agctataact ccaactgtgg acaaaagagg     660
   aatgaggtaa tgaagagcaa agaatcgcct taaagtagct ttgtccaccc taaacccccc     720
   ccaaagtcaa attacaattt cgtccccaat accagggatg acagaaaata gatttgtaat     780
   aaccgtagct ccccagaaag atatctggcc ccaggggagc acatagccca aaaaagctct     840
   ggctatgatt aaaattaaaa tcgtagagcc tacaactcaa acaggagtaa gcttatacga     900
   agaatagtaa aggccccgac ccgtatgaat gtataaacac ataaaaaata acctcgcggt     960
   gttgctatga atagctcgaa ttcctcaccc ataata                               996
<210> 4
   <211> 1115
   <212> DNA
   <213> Lepeophtheirus salmonis
<400> 4
   ctaagtggat tttgatctgg attagtgggt ttagctataa gagtcatcat ccgtcttgag      60
   ctgtctcagc cgggagcata tttaggagat tcccaggttt ataatgttat tgtaactgct     120
   catgccttca ttataatctt tttcatagta atacctgtat taattggggg gtttggaaat 180
   tgattagttc ctttaatact gggggcacct gacatagctt tcccccgctt aaacaatata 240
   agattttggt ttttaatacc ctcttcgagt ttattactta taagggcatt agtagaaagt 300
   ggtgcaggaa ctgggtgaac agtatatccc cccctgtctt cgggagtttt tcactccggg 360
   gcttcagtag attttgcgat tttttccctc cacttagctg gagtgtcttc tttattaggg 420
   gctgtaaact ttattagaac tattacaaat ttacggtgct tgggactttt agtggggcaa 480
   atgccaatat tcccctgatc cgttttaatt actgctgtgc ttttactctt gtctttacca 540
   gtgttagcgg gagcaattac tatacttctt actgatcgaa atttaaatac aacttttttt 600
   gaccctagag gtggggggga tcctatttta taccagcatt tattttgatt tttcgggcac 660
   cctgaagttt atattctaat tcttccaggg tttgggttaa tctctcagat tattacccaa 720
   gaaacttgta aagatgaggc ttttggttcg cttggtataa tttatgcaat agtagcaatt 780
   ggagtattgg ggtttattgt ttgagctcat cacatgttta cagttgggtt agactcagac 840
   actcgagcgt attttacggc agccactata gtaattgcta tccctacagg gattaaagta 900
   tttagttgat tagggacttt atatggaagt aagattattt ataccccctc aatttattgg 960
   gtggtagggt ttctgtttct ttttacagtg ggaggattaa caggtattgt attagctaac 1020
   tctgccttgg atatgacttt gcatgacact tactacgtag ttgcccactt tcactatgtt 1080
   cttagaatag gggcagtttt tgccctaata gcggg 1115
<210> 5
   <211> 1255
   <212> DNA
   <213> Lepeophtheirus salmonis
<400> 5
   caaaataatc taattattta tgtttgtgta atgcgggcag ttattacgcc tgtcaaatga      60
   cttcaaacta ataaaagttt agctcacaat ccgtctgagt aaggtaagga cagccctact     120
   ctgaagtata agacagtaaa aacttggccc aggaaaatat agtttccctc tacaggtttt     180
   gctccaatcc aggttaaaag aaaaaaaatc ccgataaaag ctcaaaacat aagtattttc     240
   gaggggttaa atgtagcagg tacggcggtt cttttatccc ccagcaaaaa tgggaaaaaa     300
   aacaaaattc ttacagacaa agctaaagcc actacccccc ctaacttatt tgggatagac     360
   cgcaaaatag cataagcaaa caaaaaatac cattcaggtt gaatgtgagc aggagttact     420
   aaaggattag ctggaataaa gttttcaggg tctgctaata atcagggaga gtaagaaact     480
   aaagctccca tgagacaaaa aataagaccg aagccaaagg cgtctttaaa ggtatagtaa     540
   gggtcaaaag gaatcttata atagtttcct ggaaccccca atggatttga ggaccctgtt     600
   gaatgaagaa aaattgtatg agctataact ccaactgtgg acaaaagagg aatgaggtaa     660
   tgaagagcaa agaatcgcct taaagtagct ttgtccaccc taaaaccccc tcaaagtcaa     720
   attacaattt cgtccccaat accagggatg acagaaaata gatttgtaat aaccgtagct     780
   cctcagaaag atatctggcc ccaggggagc acatagccca aaaaagctct ggctatgatt     840
   aaaattaaaa tcgtagagcc tacaactcaa acaggagtaa gcttatacga agaatagtaa     900
   aggccccgac ccgtatgaat gtataaacac ataaaaaata acctcgcggt gttgctatga     960
   atagctcgaa ttcctcaccc ataattaaca tcccgcatga tatggtctac ggataaaaac    1020
   gctgtagaaa tttctcttga ataatgcata gccaaaaaaa ttccggtcat aatttgggta    1080
   ataagacata gcccaagtaa agacccaaag ttccaccccg acgaaatatt aactggggtg    1140
   ggaagtgtta gaagaggatt atagatagaa agagttttat aagaattcat aaatggagct    1200
   ttctggcaga aaaagtgcgt taattttaga aattaagtat aagagattct tgtaa          1255
<210> 6
   <211> 18
   <212> DNA
   <213> Lepeophtheirus salmonis
<400> 6
   ttaaacaata taagattt 18
<210> 7
   <211> 24
   <212> DNA
   <213> Lepeophtheirus salmonis
<400> 7
   tagaaagtgg tgcaggaact gggt 24
<210> 8
   <211> 26
   <212> DNA
   <213> Lepeophtheirus salmonis
<400> 8
   gggcaccctg aagtttatat tctaat 26
<210> 9
   <211> 26
   <212> DNA
   <213> Lepeophtheirus salmonis
<400> 9
   aaccaaaagc ctcatcttta caagtt 26
<210> 10
   <211> 21
   <212> DNA
   <213> Lepeophtheirus salmonis
<400> 10
   aggttaaaga aaaaaaatcc c 21
<210> 11
   <211> 21
   <212> DNA
   <213> Lepeophtheirus salmonis
<400> 11
   taaatgtagc agtacggcgg t 21
<210> 12
   <211> 19
   <212> DNA
   <213> Lepeophtheirus salmonis
<400> 12
   gcaggtacgg cggttcttt 19
<210> 13
   <211> 28
   <212> DNA
   <213> Lepeophtheirus salmonis
<400> 13
   gctttagctt tgtctgtaag aattttgt 28
<210> 14
   <211> 27
   <212> DNA
   <213> Lepeophtheirus salmonis
<400> 14
   ttcttacaga caaagctaaa gccacta 27
<210> 15
   <211> 25
   <212> DNA
   <213> Lepeophtheirus salmonis
<400> 15
   agtaactcct gctcacattc aacct 25
<210> 16
   <211> 16
   <212> DNA
   <213> Lepeophtheirus salmonis
<400> 16
   cctcttygag tttatt 16
<210> 17
   <211> 17
   <212> DNA
   <213> Lepeophtheirus salmonis
<400> 17
   ttccagggtt tggrtta 17
<210> 18
   <211> 19
   <212> DNA
   <213> Lepeophtheirus salmonis
<400> 18
   caaaacataa gycattttc 19
<210> 19
   <211> 15
   <212> DNA
   <213> Lepeophtheirus salmonis
<400> 19
   cagcaaaaat ggraa 15
<210> 20
   <211> 14
   <212> DNA
   <213> Lepeophtheirus salmonis
<400> 20
   ccccccyaac ttat 14